(19) Europäisches Patentamt
European Patent Office
Office européen des brevets

(11) **EP 3 736 817 A1**

(12) **EUROPÄISCHE PATENTANMELDUNG**

(43) Veröffentlichungstag:
**11.11.2020 Patentblatt 2020/46**

(51) Int Cl.:
*G16H 50/70* (2018.01)   *G16H 30/40* (2018.01)

(21) Anmeldenummer: **20162867.4**

(22) Anmeldetag: **13.03.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **02.04.2019 EP 19166679**

(71) Anmelder: **Siemens Healthcare Diagnostics Inc.
Tarrytown, NY 10591 (US)**

(72) Erfinder:
• **Büttner, Florian
81541 München (DE)**

• **Geipel, Markus Michael
80799 München (DE)**
• **Marquardt, Gaby
91353 Hausen (DE)**
• **Seidel, Daniela
91083 Baiersdorf (DE)**
• **Tietz, Christoph
85521 Ottobrunn (DE)**

(74) Vertreter: **Castorph, Simon Johannes
Siemens Healthcare Diagnostics Products GmbH
P.O. Box 22 16 34
80506 München (DE)**

(54) **ÜBERPRÜFUNG UND/ODER VERBESSERUNG DER KONSISTENZ VON DATENKENNZEICHNUNGEN BEI DER MEDIZINISCHEN BILDVERARBEITUNG**

(57)    Die vorliegende Erfindung betrifft ein Computer-implementiertes Verfahren und eine Datenverarbeitungsvorrichtung zur Bereitstellung und Anwendung eines trainierten probabilistischen graphischen Modells zur Überprüfung und/oder Verbesserung der Konsistenz von Datenkennzeichnungen bei der medizinischen Bildverarbeitung, eine Verwendung des Modells zur Überprüfung und/oder Verbesserung der Konsistenz von Datenkennzeichnungen bei der medizinischen Bildverarbeitung, ein Computer-implementiertes Verfahren zur Überprüfung und/oder Verbesserung der Konsistenz von Datenkennzeichnungen bei der medizinischen Bildverarbeitung, eine Datenverarbeitungsvorrichtung die ausgebildet ist, die Konsistenz von Datenkennzeichnungen bei der medizinischen Bildverarbeitung zu überprüfen und/oder zu verbessern, sowie ein entsprechendes Computerprogrammprodukt und ein computerlesbares Medium.

FIG 2

EP 3 736 817 A1

**Beschreibung**

Die vorliegende Erfindung betrifft ein Computer-implementiertes Verfahren und eine Datenverarbeitungsvorrichtung zur Bereitstellung und Anwendung eines trainierten probabilistischen graphischen Modells zur Überprüfung und/oder Verbesserung der Konsistenz von Datenkennzeichnungen bei der medizinischen Bildverarbeitung, eine Verwendung des Modells zur Überprüfung und/oder Verbesserung der Konsistenz von Datenkennzeichnungen bei der medizinischen Bildverarbeitung, ein Computer-implementiertes Verfahren zur Überprüfung und/oder Verbesserung der Konsistenz von Datenkennzeichnungen bei der medizinischen Bildverarbeitung, eine Datenverarbeitungsvorrichtung, die ausgebildet ist, die Konsistenz von Datenkennzeichnungen bei der medizinischen Bildverarbeitung zu überprüfen und/oder zu verbessern, sowie ein entsprechendes Computerprogrammprodukt und ein computerlesbares Medium.

Die Verfügbarkeit gekennzeichneter Daten ist eine wichtige Voraussetzung für maschinelles Lernen. In der medizinischen Bildverarbeitung werden Datenkennzeichnungen typischerweise von Experten erstellt. Dieser Kennzeichnungsansatz ist aus mehreren Gründen unvorteilhaft: Zunächst ist die Aneignung von Expertenwissen ein sehr zeitaufwändiger und arbeitsintensiver Prozess, der nur nach langjähriger intensiver Praxis befriedigende Resultate liefert. Weiterhin ist das Verfahren fehleranfällig. So können die Expertenbewertungen aufgrund unterschiedlich langer Erfahrung oder aufgrund unterschiedlichen Vorwissens durchaus voneinander abweichen. Insbesondere bei der Bewertung von Zellentwicklungslinien entsprechen die Kennzeichnungen vielfach einer eher künstlichen Kategorisierung mehrerer Stufen einer kontinuierlichen Entwicklung. Hier legen Experten teilweise unterschiedliche Grenzen zwischen Entwicklungsstufen fest oder es kommt zu völligen Fehlklassifizierungen der Zellen.

Wie sich beispielsweise dem Dokument van der Meer et al., 2007, J Clin Pathol, 60(7), 838-839 entnehmen lässt, gibt es in bis zu 10% der Proben Meinungsverschiedenheiten zwischen den bewertenden Experten. Das Dokument erwähnt auch Fälle auffallender Uneinigkeit, wobei in einem Fall einer Zelle fünf verschiedene Bewertungen zugeordnet wurden und über 30% der Experten nicht in der Lage waren, vorhergehende Klassifizierungen zu reproduzieren.

Die von Experten erstellten Klassifikationen sind somit eine relativ unzuverlässige Grundlage für einen Maschinenlern-Ansatz, der auf Mustererkennung ohne Plausibilitätskontrolle basiert.

Der vorliegenden Erfindung liegt somit die Aufgabe zugrunde, ein Verfahren bereitzustellen, das Fehlbewertungen oder falsche Klassifizierungen von Zellen durch Experten vermeidet, Korrekturen von Bewertungen vorschlägt und einem morphologisch analysierenden Fachmann Feedback bezüglich der Plausibilität seiner Einschätzung gibt.

Diese Aufgabe wird durch die Gegenstände der unabhängigen Ansprüche gelöst. In den abhängigen Ansprüchen sind weitere vorteilhafte Aspekte der Erfindung reflektiert.

Die Erfindung betrifft zunächst ein Computer-implementiertes Verfahren zur Erzeugung eines trainierten probabilistischen graphischen Modell zur Überprüfung und/oder Verbesserung der Konsistenz von Datenkennzeichnungen bei der medizinischen Bildverarbeitung, wobei das Verfahren die folgenden Schritte umfasst:

- Bereitstellen eines Satzes von Bildern als Trainingsdaten, wobei die Bilder Bildinformationen jeweils einer Zelle umfassen;
- Errechnen eines Merkmalsraumes für die Trainingsdaten, vorzugsweise mit Hilfe eines Deep Convolutional Neural Network (DCNN), wobei ein Bild einem Merkmalsvektor im Merkmalsraum entspricht;
- Errechnen der Ähnlichkeiten zwischen mindestens zwei Datenpunkten der Trainingsdaten auf Basis des Merkmalsraumes;
- Bereitstellen von Experten-generierten Datenkennzeichnungen für die Zellen, deren Bilder als Trainingsdaten bereitgestellt wurden;
- Errechnen von verborgenen Datenkennzeichnungen auf Basis der Ähnlichkeiten zwischen mindestens zwei Datenpunkten der Trainingsdaten und der Experten-generierten Datenkennzeichnungen, vorzugsweise mit Hilfe eines Viterbi Algorithmus;
- Erzeugung eines probabilistischen graphischen Modells zur Anpassung der verborgenen Datenkennzeichnungen, wobei das probabilistische Modell vorzugsweise ein Conditional Random Field (CRF) Modell ist.

Dieses Verfahren ist auf nicht geordnete Zellen gerichtet. Es bezieht sich somit auf jeden Zelltyp bzw. jede phänotypische Ausprägung von Zellmerkmalen und setzt nicht voraus, dass die zu analysierenden Zellen ähnlich sind, indem sie beispielsweise aus einer inhärenten Entwicklungslinie hervorgegangen sind.

In einem alternativen Ansatz bezieht sich die vorliegende Erfindung auf ein Computer-implementiertes Verfahren zur Erzeugung eines trainierten probabilistischen graphischen Modells zur Überprüfung und/oder Verbesserung der Konsistenz von Datenkennzeichnungen bei der medizinischen Bildverarbeitung, wobei die Zelle verschiedene Entwicklungsstufen einer Zelle repräsentieren kann, umfassend die folgenden Schritte:

- Bereitstellen eines Satzes von Bildern als Trainingsdaten, wobei die Bilder Bildinformationen jeweils einer Zelle umfassen;

- Errechnen eines Merkmalsraumes für die Trainingsdaten, vorzugsweise mit Hilfe eines Deep Convolutional Neural Network (DCNN), wobei ein Bild einem Merkmalsvektor im Merkmalsraum entspricht;
- Errechnen der Ähnlichkeiten zwischen mindestens zwei Datenpunkten der Trainingsdaten auf Basis des Merkmalsraumes;
- Errechnen einer Pseudozeit, welche die Datenpunkte gemäß einer Entwicklungsabfolge ordnet;
- Bereitstellen von Experten-generierten Datenkennzeichnungen für die Zellen, deren Bilder als Trainingsdaten bereitgestellt wurden;
- Errechnen von verborgenen Datenkennzeichnungen auf Basis der in der Pseudozeit reflektierten Entwicklungsabfolge und der Experten-generierten Datenkennzeichnungen, vorzugsweise mit Hilfe eines Viterbi Algorithmus;
- Erzeugung eines probabilistischen graphischen Modells zur Anpassung der verborgenen Datenkennzeichnungen, wobei vorzugsweise das probabilistische Modell bei einer linearen Entwicklungsabfolge ein Hidden Markov Modell (HMM) und bei einer dichotomen Entwicklungsabfolge ein Hidden Markov Baum (HMT)ist.

Dieses Verfahren ist auf geordnete Zellen gerichtet. Es bezieht sich somit insbesondere auf Zellen oder phänotypische Ausprägungen von Zellmerkmalen, welche ähnlich sind und sich typischerweise aus einer inhärenten Entwicklungslinie ergeben.

Im Zentrum der vorliegenden Anmeldung werden die vorgenannten vorbereitenden Computer-implementierten Verfahren als Grundlage verwendet, um Verfahren durchzuführen, welche eine Anpassung im Sinne einer Überprüfung und/oder Verbesserung der verborgenen Datenkennzeichnungen auf Basis des probabilistischen graphischen Modells vorsehen:

So bezieht sich die vorliegende Anmeldung insbesondere und vorteilhafterweise auf ein Computer-implementiertes Verfahren, das auf Zellen allgemeiner Art bezogen ist, wobei ein probabilistische graphische Modell, vorzugsweise ein probabilistische graphische Modell wie es im hierin beschriebenen Verfahren erhalten wurde, zur Anpassung von verborgenen Datenkennzeichnungen bei der medizinischen Bildverarbeitung von Zellen verwendet wird. Dieses Verfahren umfasst die Schritte:

- Bereitstellen von verborgenen Datenkennzeichnungen auf Basis der Ähnlichkeiten zwischen mindestens zwei Datenpunkten von Bildern als Trainingsdaten und mit diesen Trainingsdaten assoziierten Experten-generierten Datenkennzeichnungen, wobei die Bilder Bildinformationen jeweils einer Zelle umfassen;
- Verknüpfen der verborgenen Datenkennzeichnungen und der Experten-generierten Datenkennzeichnungen, wobei die Verknüpfung ein Verbinden aller verborgenen Datenkennzeichnungen untereinander mittels ungerichteter Kanten und ein Verbinden von verborgenen Datenkennzeichnungen und Experten-generierten Datenkennzeichnungen mittels gerichteter Kanten umfasst;
- Anpassen oder Bestätigen der verborgenen Datenkennzeichnungen auf Basis des probabilistischen graphischen Modells

So bezieht sich die vorliegende Anmeldung weiterhin und vorteilhafterweise auf ein Computer-implementiertes Verfahren, das auf Zellen einer Entwicklungsreihe bezogene ist, wobei ein probabilistisches graphisches Modell, vorzugsweise ein probabilistisches graphisches Modell wie es im hierin beschriebenen Verfahren erhalten wurde, zur Anpassung von verborgenen Datenkennzeichnungen bei der medizinischen Bildverarbeitung von Zellen, welche verschiedene Zell-Entwicklungsstufen repräsentieren können, verwendet wird. Dieses Verfahren umfasst die Schritte

- Bereitstellen von verborgenen Datenkennzeichnungen auf Basis der Ähnlichkeiten zwischen mindestens zwei Datenpunkten eines Satzes von Bildern als Trainingsdaten, wobei die Bilder Bildinformationen jeweils einer Zelle umfassen, und einer daraus abgeleiteten Pseudozeit, sowie von mit den Trainingsdaten assoziierten Experten-generierten Datenkennzeichnungen;
- Verknüpfen der verborgenen Datenkennzeichnungen und der Experten-generierten Datenkennzeichnungen, wobei die Verknüpfung ein Verbinden aller Datenkennzeichnungen mittels gerichteter Kanten umfasst;
- Anpassen oder Bestätigen der verborgenen Datenkennzeichnungen auf Basis des probabilistischen graphischen Modells.

Ein Anpassen der verborgenen Datenkennzeichnungen kann beispielsweise eine Korrektur einer bisherigen Zuordnung oder Klassifizierung einer Zelle sein. Dies wäre notwendig, wenn es eine Diskrepanz zwischen der verborgenen Datenkennzeichnung und der Experten-generierten Datenkennzeichnungen gibt. Alternativ kann eine Bestätigung einer bisherigen Zuordnung oder Klassifizierung einer Zelle erfolgen. Dies wäre dann der Fall, wenn es keine Diskrepanz zwischen der verborgenen Datenkennzeichnung und der Experten-generierten Datenkennzeichnungen gibt, also eine identische oder plausible Klassifizierung vorliegt.

Weitere Details und konkrete Ausgestaltungen des Verfahrens lassen sich den Beispielen entnehmen, die illustrieren, wie mit Hilfe des erfindungsgemäßen Verfahrens eine bisher unerreichte Verbesserung der Datenkennzeichnungskonsistenz erreicht werden konnte.

Eine Ausführungsform des Errechnens eines Merkmalsraumes für den Trainingsdatensatz, wie in den oben beschriebenen Verfahren erwähnt, sieht die Verwendung eines Maschinenlernalgorithmus für diesen Schritt vor. Unter einem Maschinenlernalgorithmus wird im Kontext dieser Anmeldung insbesondere ein Algorithmus, der zum Maschinellen Lernen ausgebildet ist, verstanden. Maschinenlern-Algorithmen werden typischerweise in zwei Klassen unterteilt: Algorithmen des überwachten Lernens ("supervised learning") und Algorithmen des nicht-überwachten Lernens ("unsupervised learning").Das überwachte Lernen ist die Fähigkeit von AI-Systemen, Gesetzmäßigkeiten nachzubilden, wobei die Ergebnisse durch Naturbeobachtungen oder Expertenurteile bereits vorhanden sind und genutzt werden, um das System anzulernen. Repräsentative Beispiele für das überwachte Lernen sind der Bayes-Klassifikator, der Naiver Bayes-Klassifikator, die Nächste-Nachbarn-Klassifikation, die Diskriminanzanalyse sowie künstliche Neuronale Netze. Beim überwachten Lernen kann typischerweise eine Funktionenklasse verwendet werden, welche beispielsweise auf Entscheidungsbäumen ("decision trees"), einem Random Forest, einer logistischen Regression, einer Support Vector Machine, Netz, einer Kernel-Methode, oder ähnlichem oder Kombinationen davon basiert. Das unüberwachte Lernen bezeichnet maschinelles Lernen, ohne im Voraus bekannte Zielwerte zu kennen. Dabei wird versucht, aus den Eingabedaten Muster zu erkennen. Beispiele sind die automatische Segmentierung (Clustering), sowie die Komprimierung von Daten zur Dimensionsreduktion. Die Komprimierung wird beispielsweise bei der Hauptkomponentenanalyse umgesetzt. Der Maschinenlernalgorithmus kann weiterhin beispielsweise zum tiefen Lernen ("deep learning") und/oder zum bestärkendem Lernen ("reinforcement learning") und/oder zum Marginal Space Learning ausgebildet sein. Mögliche Implementierungen des Maschinenlernalgorithmus können beispielsweise Künstliche Intelligenz verwenden. Für das Optimieren können dem Fachmann bekannte Optimierungsverfahren verwendet werden. Berechnungen, insbesondere beim Optimieren, können beispielsweise mittels eines Prozessorsystems ausgeführt werden. Das Prozessorsystem kann beispielsweise einen oder mehrere Grafikprozessoren aufweisen.

Es ist besonders bevorzugt, dass das Errechnen der Merkmale mit Hilfe eines Deep Convolutional Neural Network (DCNN) durchgeführt wird. Ein Convolutional Neural Network oder faltendes neuronales Netzwerk, ist ein künstliches neuronales Netz, das prinzipiell aus einem oder mehreren Convolutional Layers, gefolgt von einem Pooling Layer besteht. Ein DCNN, wie vorzugsweise im Rahmen der vorliegenden Erfindung verwendet, enthält mehrere Wiederholungen dieser Schichten. Der Convolutional Layer umfasst typischerweise eine zwei- oder dreidimensionale Matrix. Die Aktivität jedes Neurons wird über eine diskrete Faltung berechnet. Ein Neuron in dieser Schicht reagiert nur auf Reize aus seiner lokalen Umgebung. Im Pooling Layer werden im Prinzip überflüssige Informationen verworfen. Eine beispielhafte Umsetzung des Pooling ist das Max-Pooling, bei dem aus jedem 2 x 2 Quadrat aus Neuronen des Convolutional Layers nur die Aktivität des aktivsten Neurons für die weiteren Rechenschritte beibehalten wird. Nach einigen sich wiederholenden Einheiten, bestehend aus Convolutional und Pooling Layer, kann das Netzwerk mit einem oder mehreren Fully-connected Layer abschließen. Die Ausgabe der letzten Schicht des DCNN wird typischerweise durch eine Softmax-Funktion, d.h. einer translationsinvarianten Normalisierung über alle Neuronen im letzten Layer, in eine Wahrscheinlichkeitsverteilung überführt.

Zur Errechnung der Ähnlichkeiten zwischen zwei Datenpunkten der Trainingsdaten wird beispielsweise eine Metrik, eine Abstandsfunktion oder ein Kern (Kernel) verwendet. Dabei werden mindestens die beiden Datenpunkte als Eingabewerte verwendet, um als Ausgabewert einen positiven Wert zu erhalten, welcher den Abstand zwischen den Werten beschreibt, wobei die Ähnlichkeit zwischen den Datenpunkten einem reziproken Wert daraus entspricht. In bestimmten Ausführungsformen können zur Errechnung der Ähnlichkeiten nicht nur die Informationen aus den genannten beiden Datenpunkten, sondern ebenfalls Informationen von anderen, vorzugsweise von allen anderen Datenpunkten aus dem Datensatz verwendet werden. Ein Beispiel für einen typischerweise verwendeten Kernel ist

$$K(\mathbf{x}, \mathbf{x}') = \exp\left(-\frac{\|\mathbf{x} - \mathbf{x}'\|^2}{2\sigma^2}\right)$$

.

Dabei kann zur weiteren Bestimmung der Ähnlichkeiten zwischen zwei Datenpunkten der Trainingsdaten auf Basis des Merkmalsraumes, beispielsweis von zwei Bilddaten, vorteilhafterweise ein Ähnlichkeitsgraph erzeugt werden. Dabei werden Ähnlichkeitsgruppen graphisch zugeordnet. Dieses Verfahren ist typischerweise nicht auf Klassifikationsvorwissen angewiesen und somit frei von einer Fehlklassifikationsbeeinflussung.

Trainingsdaten für die Verfahren können Bilder umfassen, wobei die Bilder Bildinformationen jeweils einer Zelle umfassen. Es wird somit mindestens ein Satzes von Bildern als Trainingsdaten bereitgestellt, wobei die Bilder Bildinformationen jeweils einer Zelle umfassen. Die entsprechenden Informationen können in unterschiedlichen Auflösungen vorliegen. Die Trainingsdaten in Form von Bildern und zugehörigen Bildinformationen können ebenfalls qualitative Ergänzungen enthalten. So können sie Informationen zur Identität der Zellen enthalten. Diese Informationen können den Experten-generierten Datenkennzeichnungen entsprechen. In alternativen Ausführungsformen können die Experten-generierten Datenkennzeichnungen in einem separaten Datensatz enthalten sein. In dieser Konstellation können die Bilddatensätze oder -informationen und die Datenkennzeichnungsdatensätze oder - informationen verlinkt oder mit einander referenziert sein. Die Trainingsdaten können darüber hinaus weitere Dateneinheiten umfassen, beispielsweise Informationen zur Aufnahmeeinheit, mit welcher sie erzeugt wurden, Informationen zum Zeitpunkt der Erzeugung, optional ebenfalls Patienteninformationen, geographische Informationen oder Informationen zu verwendeten Färbungsverfahren o.ä., sollten diese durchgeführt worden sein.

Die Experten-generierten Datenkennzeichnungen können Informationen zum Zelltyp, zu einer zugrundeliegenden Erkrankung, zum Status der Zellen hinsichtlich der Zellteilungsphase, beispielsweise, ob sich eine Zelle in einer G-, S- oder M-Phase des Zellzyklus befindet, enthalten. Die Datenkennzeichnungen würden in dieser besonderen Ausführungsform nicht eine inhärente Ordnung oder geordneten Zuordnung der Zellen reflektieren.

Für die Verwendung in Verfahren zur Zellklassifizierung von Zellen verschiedener Entwicklungsstufen enthalten diese Datenkennzeichnungen typischerweise Informationen zur Einschätzung bzw. Identifizierung der Entwicklungsstufe einer Zelle. Die entsprechenden Informationen können beispielsweise in Form einer Klasseneinteilung oder Entwicklungseinteilung vorliegen und somit eine inhärente Ordnung reflektieren. Die Datenkennzeichnungen können weiterhin Informationen bzgl. einer bereits vorgenommenen Korrektur und/oder einer Bestätigung der Experteneinschätzung enthalten.

Neben den Experten-generierten Datenkennzeichnungen, welche für die Verfahren aus externen Quellen bereitgestellt werden, werden verborgene Datenkennzeichnungen gemäß den beschriebenen Verfahren errechnet. Derartige verborgene Datenkennzeichnungen reflektieren Bewertungen der Zellen hinsichtlich der analysierten Parameter und basieren im Prinzip auf Merkmalsvergleichsoperationen der Trainingsdaten mit Bildinformationen zu den Zellen. Die verborgenen Datenkennzeichnungen können mit den entsprechenden, d.h. zu denselben Zellen vorhandenen, Experten-generierten Datenkennzeichnungen assoziiert sein. Hier können ebenfalls Diskrepanzen zwischen den Datenkennzeichnungen registriert und gespeichert werden.

Im Rahmen der Verwendung von Bilddaten zu Zellen verschiedener Entwicklungsstufen, welche einer inhärenten Ordnung folgen, wird eine Pseudozeit im Rahmen der Operationen im Merkmalsraum errechnet. Die Pseudozeit ist ein Konzept, das für Transitionen in biologischen Systemen entwickelt wurde. Hierbei werden individuelle Zellen während der Transition von einem Stadium zum nächsten verfolgt. Da Zellen diese Stadienwechsel typischerweise nicht in einer festgelegten Frequenz durchlaufen, kommt es zu Variationen in der Zeitdimension. Die Pseudozeit wird hierbei als verborgene, d.h. nicht beobachtete Dimension verstanden, welche den Fortschritt der Zellen während der Transitionsvorgänge beschreibt. Weitere Informationen dazu können beispielsweise aus geeigneten Literaturreferenzen wie Reid und Wernisch, 2016, Bioinforamtics, 32(19), 2973-2980 entnommen werden.

Probabilistische graphische Modelle (PGM) wie in den oben genannten Verfahren erhalten bzw. verwendet, sind im Allgemeinen Graphen, deren Knoten Zufallsvariablen sind und in denen die Abwesenheit von Kanten zwischen diesen Knoten deren Unabhängigkeit anzeigt. Die PGM stellen somit einen Formalismus bereit, der es ermöglicht, weitere probabilistische Modelle darzustellen oder umzusetzen.

Eine bevorzugte Ausführungsform des herein beschriebenen Verfahrens, welches auf der Auswertung von Zellen ohne inhärente Ordnung basiert, sieht vor, dass das probabilistische Modell ein Conditional Random Field (CRF) Modell ist. Ein CRF ist ein Typ von ungerichtetem probabilistischem Modell. Typischerweise wird es zur Segmentierung von Sequenzen verwendet. Beispielsweise würde das CRF eine Sequenz x als Eingabe erhalten und eine gleich lange Sequenz y ausgeben. Dabei kann das CRF an jeder Stelle auf die komplette Information der Eingabesequenz zugreifen, was die Verwendung von komplexen Merkmalsmengen erlaubt.

Im Rahmen der Verwendung des CRF wird dabei das Verknüpfen der errechneten Datenkennzeichnung und der Experten-generierten Datenkennzeichnung auf Basis der bedingten Wahrscheinlichkeit der Korrektheit der Experten-generierten Datenkennzeichnung durchgeführt. Dies kann mit verschiedenen Algorithmen umgesetzt werden. Beispielsweise kann auf den Algorithmus Loopy belief propagation, Alpha expansion, Mean field inference oder Linear programming relaxations zurückgegriffen werden.

Eine bevorzugte Ausführungsform des hier beschriebenen Verfahrens, welches auf der Auswertung von Zellen mit inhärenter Ordnung basiert, sieht die Verwendung von Hidden Markov Konzepten vor. Folgen die Zellen beispielsweise einer linearen Entwicklungsabfolge, ist das probabilistische Modell ein Hidden Markov Modell (HMM). Folgen andererseits die Zellen einer dichotomen Entwicklungsabfolge, ist das probabilistische Modell ein Hidden Markov Baum (Hidden Markov Tree (HMT)).

Ein HMM ist ein stochastisches Modell, in dem ein System durch eine Markov-Kette mit unbeobachteten Zuständen modelliert wird. Bei der Modellierung als Markov-Kette geht das System typischerweise auf zufällige Weise von einem Zustand in einen anderen über, wobei die Übergangswahrscheinlichkeiten nur jeweils vom aktuellen Zustand abhängen. Hierbei werden diese Zustände nicht extern beobachtet und sind somit verborgen. Jedem dieser verborgenen Zustände sind dabei beobachtbare Ausgabesymbole (Emissionen) zugeordnet, die je nach Zustand mit gewissen Wahrscheinlichkeiten auftreten. Ein HMM kann als gerichtetes Modell für sequentielle Daten verwendet werden. Dabei greift das HMM nur auf die aktuelle Eingabe zu, nicht jedoch auf die komplette Information der Eingabesequenz.

Ein Hidden Markov Baum (HMT) ist eine Abwandlung des HMM, wobei die unbeobachteten Zustände in Baumstruktur von einander abhängen bzw. aufeinander folgen. Ein Beispiel, wie ein solcher Algorithmus eingesetzt werden kann, lässt sich dem Dokument Kondo et al., Proceedings of the Eighth Workshop on Statistical Machine Translation, 2013, 503-511, Sofia, Bulgaria entnehmen. Hier wird davon ausgegangen, dass die Ausrichtungsvariablen des Algorithmus eine Baumstruktur haben, die zum Zielabhängigkeitsbaum isomorph ist. Der Algorithmus modelliert die Wahrscheinlichkeit der Verzerrung (distortion probability) basierend auf dem Quell-Abhängigkeitsbaum.

Im Rahmen der Verwendung des HMM oder des Hidden Markov Baumes (HMT) wird ebenfalls das Verknüpfen der errechneten Datenkennzeichnung und der Experten-generierten Datenkennzeichnung auf Basis der bedingten Wahrscheinlichkeit der Korrektheit der Experten-generierten Datenkennzeichnung durchgeführt. Dabei wird vorzugsweise auf den Viterbi Algorithmus zurückgegriffen.

Die Erfindung betrifft ferner eine Datenverarbeitungsvorrichtung zur Erzeugung eines trainierten probabilistischen graphischen Modells zur Überprüfung und/oder Verbesserung der Konsistenz von Datenkennzeichnungen bei der medizinischen Bildverarbeitung, beispielsweise ausgehend von ungeordneten Zellen, umfassend:

- eine Einheit zum Bereitstellen eines Satzes von Bildern als Trainingsdaten, wobei die Bilder Bildinformationen jeweils einer Zelle umfassen;
- eine Einheit zum Errechnen eines Merkmalsraumes für die Trainingsdaten;
- eine Einheit zur Errechnung der Ähnlichkeiten zwischen mindestens zwei Datenpunkten auf Basis des Merkmalsraumes;
- eine Einheit zum Bereitstellen von Experten-generierten Datenkennzeichnungen für die Zellen, deren Bilder als Trainingsdaten bereitgestellt wurden;- eine Einheit zur Errechnung von verborgenen Datenkennzeichnungen auf Basis (i) der Ähnlichkeiten zwischen mindestens zwei Datenpunkten oder auf Basis der in der Pseudozeit reflektierten Entwicklungsabfolge und (ii) der Experten-generierten Datenkennzeichnungen;
- eine Einheit zur Erzeugung eines probabilistischen graphischen Modells zur Anpassung der verborgenen Datenkennzeichnungen.

Die "Anpassung" der verborgenen Datenkennzeichnung kann beispielsweise die Form eines Verknüpfens einer Datenkennzeichnung mit dem Bild haben, wobei das Verknüpfen eine Korrektur oder Bestätigung der bereits vorhandenen Experten-generierten Datenkennzeichnung für dieses Bild umfasst, also eine Überprüfung und/oder Verbesserung dieser Experten-generierten Datenkennzeichnung, sowie die Ausgabe und/oder Speicherung des Bildes zusammen mit der korrigierten oder bestätigten verknüpften Datenkennzeichnung.

Die Erfindung betrifft ferner eine Datenverarbeitungsvorrichtung zur Erzeugung eines trainierten probabilistischen graphischen Modells zur Überprüfung und/oder Verbesserung der Konsistenz von Datenkennzeichnungen bei der medizinischen Bildverarbeitung von Zellen, welche beispielsweise verschiedene Entwicklungsstufen einer Zelle repräsentieren und damit einer inhärenten Ordnung unterliegen, umfassend:

- eine Einheit zum Bereitstellen eines Satzes von Bildern als Trainingsdaten, wobei die Bilder Bildinformationen jeweils einer Zelle umfassen;
- eine Einheit zum Errechnen eines Merkmalsraumes für die Trainingsdaten;
- eine Einheit zur Errechnung der Ähnlichkeiten zwischen mindestens zwei Datenpunkten auf Basis des Merkmalsraumes;
- eine Einheit zur Errechnung einer Pseudozeit, welche die Datenpunkte gemäß einer Entwicklungsabfolge ordnet;
- eine Einheit zum Bereitstellen von Experten-generierten Datenkennzeichnungen für die Zellen, deren Bilder als Trainingsdaten bereitgestellt wurden;
- eine Einheit zur Errechnung von verborgenen Datenkennzeichnungen auf Basis (i) der in der Pseudozeit reflektierten Entwicklungsabfolge und (ii) der Experten-generierten Datenkennzeichnungen;
- eine Einheit zur Erzeugung eines probabilistischen graphischen Modells zur Anpassung der verborgenen Datenkennzeichnungen.

Weiterhin sieht die Erfindung eine abgewandelte Datenverarbeitungsvorrichtung vor, welche zur Anpassung von verborgenen Datenkennzeichnungen bei der medizinischen Bildverarbeitung von Zellen, beispielsweise von ungeordneten Zellen, auf Basis eines trainierten probabilistisches graphischen Modells verwendet werden kann, wobei die Datenverarbeitungsvorrichtung umfasst:

- eine Einheit zum Bereitstellen von verborgenen Datenkennzeichnungen auf Basis der Ähnlichkeiten zwischen mindestens zwei Datenpunkten eines Satzes von Bildern als Trainingsdaten und mit diesen Trainingsdaten assoziierten Experten-generierten Datenkennzeichnungen, wobei die Bilder Bildinformationen jeweils einer Zelle umfassen;

- eine Einheit zum Verknüpfen der verborgenen Datenkennzeichnungen und der Experten-generierten Datenkennzeichnungen, wobei die Verknüpfung ein Verbinden aller verborgenen Datenkennzeichnungen untereinander mittels ungerichteter Kanten und ein Verbinden von verborgenen Datenkennzeichnungen und Experten-generierten Datenkennzeichnungen mittels gerichteter Kanten umfasst;
- eine Einheit zum Erhalten von angepassten verborgenen Datenkennzeichnungen auf Basis des probabilistischen graphischen Modells.

Weiterhin sieht die Erfindung eine weitere abgewandelte Datenverarbeitungsvorrichtung vor, welche zur Anpassung von verborgenen Datenkennzeichnungen bei der medizinischen Bildverarbeitung von Zellen, welche beispielsweise verschiedene Entwicklungsstufen einer Zelle repräsentieren und damit einer inhärenten Ordnung unterliegen, auf Basis eines trainierten probabilistisches graphischen Modells verwendet werden kann, wobei die Datenverarbeitungsvorrichtung umfasst:

- eine Einheit zum Bereitstellen von verborgenen Datenkennzeichnungen auf Basis der Ähnlichkeiten zwischen mindestens zwei Datenpunkten eines Satzes von Bildern als Trainingsdaten, wobei die Bilder Bildinformationen jeweils einer Zelle umfassen, und einer daraus abgeleiteten Pseudozeit, sowie von mit den Trainingsdaten assoziierten Experten-generierten Datenkennzeichnungen;
- eine Einheit zum Verknüpfen der verborgenen Datenkennzeichnungen und der Experten-generierten Datenkennzeichnungen, wobei die Verknüpfung ein Verbinden aller Datenkennzeichnungen mittels gerichteter Kanten umfasst;
- eine Einheit zum Erhalten von angepassten verborgenen Datenkennzeichnungen auf Basis des probabilistischen graphischen Modells.

Die Erfindung betrifft ferner insbesondere eine Verwendung eines trainierten probabilistischen graphischen Modells, welches gemäß einem Verfahren zur Erzeugung eines trainierten probabilistischen graphischen Modells nach einem oder mehreren Aspekten dieser Erfindung bereitgestellt wurde, zur Überprüfung und/oder Verbesserung der Konsistenz von Datenkennzeichnungen bei der medizinischen Bildverarbeitung von Zellen.

Die erhaltenen probabilistischen Modelle decken dabei Diskrepanzen zwischen den errechneten verborgenen Datenkennzeichnungen und den Experten-generierten Datenkennzeichnungen auf, welche (i) eine Überprüfung der Experten-generierten Datenkennzeichnungen oder des Analyseverfahrens durch die Experten nahelegt, oder (ii) zu einer Korrektur der Experten-generierten Datenkennzeichnungen führt und somit die Einheitlichkeit der Klassifizierung oder Bewertung von Zellen erhöht.

Insbesondere wird das trainierte probabilistische graphische Modell verwendet, um auf Basis einer abgeleiteten verborgenen Datenkennzeichnung eine Experten-generierte Datenkennzeichnung von Bildinformationen zu korrigieren oder zu bestätigen.

Vorzugsweise wird ein erfindungsgemäßes trainiertes probabilistisches graphisches Modell wie hierin beschrieben verwendet, um auf Basis einer abgeleiteten verborgenen Datenkennzeichnung eine Experten-generierte Datenkennzeichnung von Bildinformationen zu korrigieren oder zu bestätigen. Die Verwendung von probabilistischen graphischen Modellen wie hier beschrieben zur Überprüfung und möglichen Verbesserung der Konsistenz von Datenkennzeichnungen bei der medizinischen Bildverarbeitung ist besonders vorteilhaft, da dadurch eine Überarbeitung bzw. Verbesserung von Experten-generierten Datenkennzeichnungen möglich wird und so unabhängig aufgefundene Diskrepanzen in der Bewertung einer erneuten Prüfung durch Experten zugeführt werden können. Dieses Vorgehen kann ein- oder mehrfach wiederholt werden, sodass eine weitere Optimierung und Konsistenzsteigerung umsetzbar ist.

Die Erfindung betrifft ferner ein Computer-implementiertes Verfahren zur Überprüfung und/oder Verbesserung der Konsistenz von Datenkennzeichnungen bei der medizinischen Bildverarbeitung, umfassend die Schritte:

- Bereitstellen des Bildes einer Zelle;
- Bereitstellen einer vorhandenen Experten-generierten Datenkennzeichnung;
- Errechnen eines Merkmalsraumes für das Bild;
- Anwenden eines Verfahrens auf Basis eines trainierten probabilistischen graphischen Modells auf die errechneten Merkmale des Bildes;
- Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
- Ausgabe und/oder Speicherung des Bildes zusammen mit der korrigierten oder bestätigten verknüpften Datenkennzeichnung.

In einer Ausführungsform der Erfindung sind die in dem genannten Computer-implementierten Verfahren verwendeten trainierten probabilistischen graphische Modelle jene, welche oben beschrieben sind. Zusätzlich oder alternativ können die anzuwendenden Verfahren jene Verfahren sein, welche oben beschrieben sind.

In einer spezifischen Ausführungsform bezieht sich die Erfindung auf ein Computer-implementiertes Verfahren zur Überprüfung und/oder Verbesserung der Konsistenz von Datenkennzeichnungen bei der medizinischen Bildverarbeitung umfassend die Schritte:

- Bereitstellen des Bildes einer Zelle;
- Bereitstellen einer vorhandenen Experten-generierten Datenkennzeichnung;
- Verwenden von Hintergrundinformationen zum Bild der Zelle, um die Topologie eines Hidden Markov Baumes (HMT), eine Startwahrscheinlichkeit und eine Emissionsmatrix zu definieren;
- Lernen der Merkmalsdarstellungen des Bildes unter Verwendung eines neuronales Netzwerkes;
- Wählen eines geeigneten Pseudozeit-Inferenzalgorithmus und Berechnen der Pseudozeiten anhand der Merkmalsvektoren;
- Sortieren der Experten-generierten Datenkennzeichnungen nach aufsteigender Pseudozeit;
- Einrichten eines Hidden Markov Baumes (HMT), bei dem die sortierten Experten-generierten Datenkennzeichnungen die beobachteten Informationen sind;
- Lernen der Parameter von Übergangsmatrizen unter Verwendung des verallgemeinerten EM-Algorithmus;
- Anwenden des verallgemeinerten Viterbi-Algorithmus, um die wahrscheinlichsten wahren Datenkennzeichnungen abzuleiten;
- Identifizieren von Bildern mit inkonsistenten Datenkennzeichnungen, indem die tatsächlichen Datenkennzeichnungen mit den Experten-generierten Datenkennzeichnungen verglichen werden;
- Ausgabe von Bildern mit inkonsistenten Datenkennzeichnungen, die zusätzlich vorgeschlagene Datenkennzeichnungen enthalten.

Weitere Details und Definitionen zu diesem Verfahren können den Beispielen 1 bis 4, insbesondere Beispiel 4 entnommen werden.

In einer weiteren Ausführungsform der Erfindung wird das Computer-implementierte Verfahren wie beschrieben dadurch ergänzt, dass eine Korrektur einer bereits vorhandenen Experten-generierten Datenkennzeichnung zu einer Feedbackanfrage bei einem Experten bezüglich der Datenkennzeichnungsdiskrepanz führt. Damit wird einerseits dem Experten eine Plausibilitätswarnung hinsichtlich seiner Bewertung zugespielt. Gleichzeitig kann die Feedbackgenerierung, ggf. nach einer Neubewertung der Einschätzung durch den Experten oder eine Gruppe von Experten, zu einer Verbesserung des verwendeten Modells oder zur Verbesserung eines abgeleiteten automatisierten Klassifizierungsansatzes führen.

In einer weiteren, besonders bevorzugten Ausführungsform wird das Computer-implementierte Verfahren zur Überprüfung und/oder Verbesserung der Konsistenz von Datenkennzeichnungen bei der medizinischen Bildverarbeitung, welches die Schritte umfasst:

- Bereitstellen des Bildes einer Zelle;
- Bereitstellen einer vorhandenen Experten-generierten Datenkennzeichnung;
- Errechnen eines Merkmalsraumes für das Bild;
- Anwenden eines Verfahrens auf Basis eines trainierten probabilistischen graphischen Modells auf die errechneten Merkmale des Bildes;
- Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
- Ausgabe und/oder Speicherung des Bildes zusammen mit der korrigierten oder bestätigten verknüpften Datenkennzeichnung; mindestens einmal, oder mehrmals erneut durchlaufen.

In einer weiteren Ausführungsform werden ein oder mehrere bisher nicht analysierte Bilder mit einem bereits errechneten Merkmalsraumes wie hierin beschrieben, sowie einem trainierten probabilistischen graphischen Modells wie hierin beschrieben, abgeglichen, um bei Übereinstimmungen Datenkennzeichnungen abzuleiten, vorzugsweise in dem bereits berechneten Übergangsgrenzen innerhalb des Merkmalsraumes verwendet werden.

Die Erfindung bezieht sich weiterhin auf eine Datenverarbeitungsvorrichtung, die ausgebildet ist, die Konsistenz von Datenkennzeichnungen bei der medizinischen Bildverarbeitung zu überprüfen und/oder zu verbessern, wobei die Vorrichtung mindestens einen Prozessor und einen Speicher aufweist, wobei der mindestens eine Prozessor eingerichtet ist, um einen Programmcode aus dem Speicher zu laden und auszuführen und basierend auf dem Ausführen des Programmcodes die folgenden Schritte auszuführen:

- Bereitstellen des Bildes einer Zelle;

- Bereitstellen einer vorhandenen Experten-generierten Datenkennzeichnung;
- Errechnen eines Merkmalsraumes für das Bild;
- Anwenden eines Verfahrens auf Basis eines trainierten probabilistischen graphischen Modells auf die errechneten Merkmale des Bildes;
- Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
- Ausgabe und/oder Speicherung des Bildes zusammen mit der korrigierten oder bestätigten verknüpften Datenkennzeichnung.

In einer Ausführungsform der Erfindung sind die in der genannten Datenverarbeitungsvorrichtung auszuführenden Schritte dadurch spezifiziert, dass die genannten trainierten probabilistischen graphischen Modelle jene sind, welche oben beschrieben sind. Zusätzlich oder alternativ können die genannten anzuwendenden Verfahren jene Verfahren sein, welche oben beschrieben sind.

Die Datenverarbeitungsvorrichtung wie hier beschrieben und/oder eine oder mehrere Komponenten davon können von einem Datenverarbeitungssystem gebildet sein. Das Datenverarbeitungssystem kann beispielsweise eine oder mehrere Komponenten in Form von Hardware und/oder eine oder mehrere Komponenten in Form von Software aufweisen.

Das Datenverarbeitungssystem kann beispielsweise zumindest teilweise von einem Cloud-Computing-System gebildet sein. Das Datenverarbeitungssystem kann beispielsweise ein Cloud-Computing-System, ein Computernetzwerk, ein Computer, ein Tablet-Computer, ein Smartphone oder ähnliches oder eine Kombination davon sein und/oder aufweisen.

Die Hardware kann beispielsweise mit einer Software zusammenwirken und/oder mittels einer Software konfigurierbar sein. Die Software kann beispielsweise mittels der Hardware ausgeführt werden. Bei der Hardware kann es sich beispielsweise um ein Speichersystem, ein FPGA-System (Fieldprogrammable gate array), ein ASIC-System (Applicationspecific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen. Insbesondere kann eine Komponente der Datenverarbeitungsvorrichtung nach einem der Aspekte, die in dieser Anmeldung offenbart sind, welche dazu ausgebildet ist, einen gegebenen Schritt eines Verfahrens nach einem der Aspekte, die in dieser Anmeldung offenbart sind, auszuführen, in Form einer Hardware implementiert sein, welche zum Ausführen des gegebenen Schritts konfiguriert ist und/oder welche zum Ausführen einer computerlesbaren Anweisung derart konfiguriert ist, dass die Hardware mittels der computerlesbaren Anweisung zum Ausführen des gegebenen Schritts konfigurierbar ist. Insbesondere kann das System einen Speicherbereich, beispielsweise in Form eines computerlesbaren Mediums, aufweisen, in welchem computerlesbare Anweisungen, beispielsweise in Form eines Computerprogramms, gespeichert sind.

Ein Datentransfer zwischen Komponenten des Datenverarbeitungssystems kann beispielsweise jeweils mittels einer geeigneten Datentransfer-Schnittstelle erfolgen. Die Datentransfer-Schnittstelle zum Datentransfer an und/oder von einer Komponente des Datenverarbeitungssystems kann zumindest teilweise in Form von Software und/oder zumindest teilweise in Form von Hardware realisiert sein. Die Datentransfer-Schnittstelle kann beispielsweise zum Abspeichern von Daten in und/oder zum Laden von Daten aus einem Bereich des Speichersystems ausgebildet sein, wobei auf diesen Bereich des Speichersystems eine oder mehrere Komponenten des Datenverarbeitungssystems zugreifen können.

Die Erfindung bezieht sich weiterhin auf ein Computerprogrammprodukt mit einem Computerprogramm, welches direkt in eine Speichereinrichtung eines Computers ladbar ist, mit Programmabschnitten, um alle Schritte eines Verfahrens nach einem oder mehreren der hierin genannten Aspekte auszuführen, wenn das Computerprogramm in dem Computer ausgeführt wird.

Das Computerprogramm ist in das Speichersystem des Datenverarbeitungssystems ladbar und von dem Prozessorsystem des Datenverarbeitungssystems ausführbar. Das Datenverarbeitungssystem kann beispielsweise mittels des Computerprogramms derart ausgebildet sein, dass das Datenverarbeitungssystem die Schritte eines Verfahrens nach einer der Ausführungsformen, die in dieser Anmeldung genannte sind, ausführen kann, wenn das Computerprogramm von dem Datenverarbeitungssystem ausgeführt wird.

Das Computerprogrammprodukt kann beispielsweise das Computerprogramm sein oder neben dem Computerprogramm mindestens einen zusätzlichen Bestandteil umfassen. Der mindestens eine zusätzliche Bestandteil des Computerprogrammprodukts kann als Hardware und/oder als Software ausgebildet sein. Das Computerprogrammprodukt kann beispielsweise ein Speichermedium, auf dem zumindest ein Teil des Computerprogrammprodukts gespeichert ist, und/oder ein Schlüssel zur Authentifizierung eines Benutzers des Computerprogrammprodukts, insbesondere in Form eines Dongles, aufweisen.

Das Computerprogrammprodukt und/oder das Computerprogramm kann beispielsweise ein Cloud-Anwendungs-Programm aufweisen, welches zum Verteilen von Programmabschnitten des Computerprogramms auf verschiedene Verarbeitungseinheiten, insbesondere verschiedene Computer, eines Cloud-Computing-Systems ausgebildet ist, wobei jede der Verarbeitungseinheiten zum Ausführen eines oder mehrerer Programmabschnitte des Computerprogramms ausgebildet ist.

Die Erfindung bezieht sich weiterhin auf ein computerlesbares Medium, auf welchem von einem Computer einlesbare und ausführbare Programmabschnitte gespeichert sind, um alle Schritte eines Verfahrens nach einem oder mehreren der hierin genannten Aspekte auszuführen, wenn die Programmabschnitte von dem Computer ausgeführt werden.

Auf dem computerlesbaren Medium kann beispielsweise das Computerprogrammprodukt nach einer der Ausführungsformen, die in dieser Anmeldung offenbart sind, und/oder das Computerprogramm nach einer der Ausführungsformen, die in dieser Anmeldung offenbart sind, gespeichert sein.

Das computerlesbare Medium kann beispielsweise ein Memorystick, eine Festplatte oder ein sonstiger Datenträger sein, der insbesondere lösbar mit dem Datenverarbeitungssystem verbunden oder fest in das Datenverarbeitungssystem integriert sein kann. Das computerlesbare Medium kann beispielsweise einen Bereich des Speichersystems des Datenverarbeitungssystems bilden.

Die erfindungsgemäße Lösung im Kontext der oben beschriebenen Aspekte ermöglicht somit in einem Nebenaspekt die Verbesserung eines ebenfalls hierin enthaltenen allgemeinen Klassifizierungsverfahrens zur Zellzuordnung oder Zelleinteilung, welches auf einem simplen Maschinenlernalgorithmus ohne Plausibilitätskontrolle basiert. Dabei wird vor allem die Datenqualität erhöht, sodass eine genauere und damit auch kosten-effizientere automatische Klassifizierung umsetzbar wird. Darüber hinaus ermöglichen einige der hierin genannten erfindungsgemäßen Gegenstände die Festlegung von konsistenteren und reproduzierbareren Grenzen zwischen Zellentwicklungsstufen. Dabei werden die Experten-generierten Datenkennzeichnungen konsolidiert und somit breiter einsetzbar. Weiterhin kann die Feedback-Generierung, wie oben ausgeführt, zur Erstellung neuer Richtlinien oder Regeln zur manuellen Bewertung beitragen. Darüber hinaus kann die erfindungsgemäße Lösung auch zu einer Verbesserung des Dialogs zwischen dem Experten und einem Maschinenlern-Ingenieur führen, was zu einer Verbesserung der Datenkonsistenz beiträgt.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander getrennt sind. Der Ausdruck "basierend auf" oder "auf Basis von" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von weiteren Ausführungsbeispielen unter Hinweis auf die beigefügten Figuren erläutert. Die Darstellung in den Figuren ist schematisch, stark vereinfacht und nicht zwingend maßstabsgetreu.

Es zeigen:

**Figur** 1 den Status Quo des maschinellen Lernprozesses aus dem Stand der Technik.

**Figur** 2 ein schematisches Berechnungsverfahren auf Basis ungeordneter Zellen.

**Figur** 3 die Verknüpfung von Datenkennzeichnungen für ungeordnete Zellen.

**Figur** 4 die Entwicklungsstadien eines Granulozyten. Von links nach rechts sind die Stadien Myeloblast, Promyelozyt, Myelozyt, Metamyelozy und stabkerniger neutrophiler segmentkerniger Granulozyt dargestellt.

**Figur** 5 ein schematisches Berechnungsverfahren für Zellen, welche einer Transition von Zellentwicklungsstadien unterliegen.

**Figur** 6 ein Deep Convolutional Neural Network für die Berechnung eines Merkmalsraumes für Trainingsdatensätze.

**Figur** 7 die Darstellung von Zellen im Merkmalsraum. Die abgeleiteten Informationen zu den Zellen sind in Form eines Ähnlichkeitsgraphen angeordnet, wobei der Kurvenverlauf im Verhältnis zum Entwicklungsstadium der Zellen verläuft.

**Figur** 8 die Reihenfolge von Zellen in der Pseudozeit, wobei eine Verknüpfung zwischen der Pseudozeit und der Repräsentation gem. Bilddaten hergestellt werden kann.

**Figur** 9 die Verknüpfung von verborgenen Datenkennzeichnungen und Experten-generierten Datenkennzeichnungen als Ausgangssituation für die Anwendung eines Hidden Markov Models.

**Figur 10** die Verknüpfung von verborgenen Datenkennzeichnungen und Experten-generierten Datenkennzeichnungen nach Anwendung eines Hidden Markov Models.

**Figur 11** Diskrepanzen zwischen den verborgenen Datenkennzeichnungen und den Experten-generierten Datenkennzeichnungen welche zur Aufdeckung von Fehlbewertungen führen können.

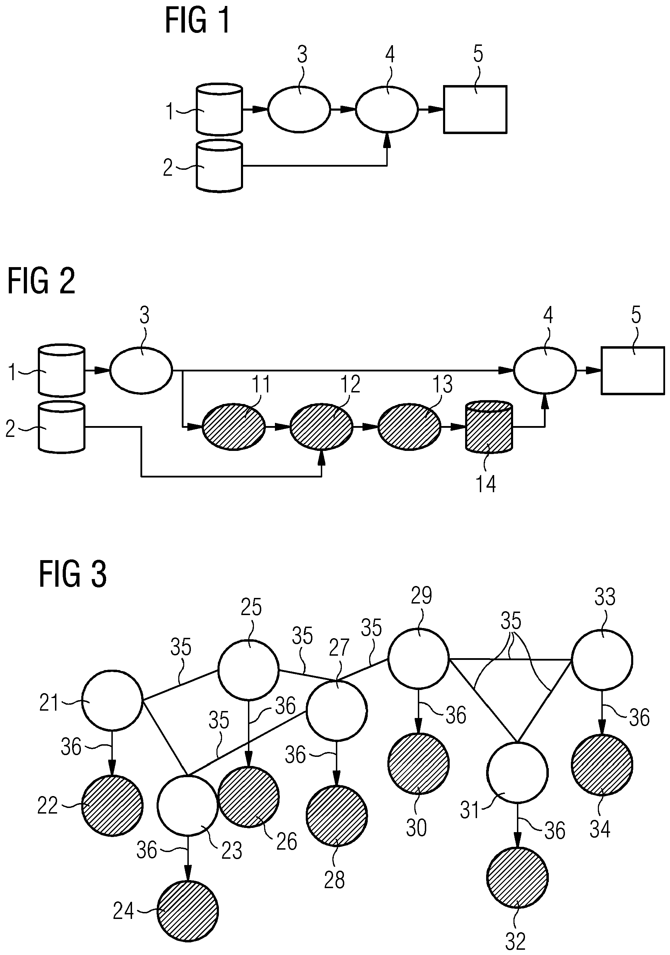

**Figur 1** zeigt den Status Quo des maschinellen Lernprozesses aus dem Stand der Technik. Dabei wird mit Bilddaten **1** eine Merkmalsberechnung **3** durchgeführt. Die Ergebnisse werden einem Modul für Maschinenlernen **4** zugeführt. Diesem Modul werden ebenfalls Experten-generierte Datenkennzeichnungen **2** zugeführt. Nach Durchlaufen eines Maschinenlernprozesses **4** kann ein Klassifizierungsmodell **5** erstellt werden.

**Figur 2** zeigt ein erfindungsgemäßes schematisches Berechnungsverfahren auf Basis ungeordneter Zellen bzw. willkürlicher Klassen. Dabei wird mit Bilddaten **1** zunächst eine Merkmalsberechnung **3** durchgeführt. Während die bisherigen Verfahren die Ergebnisse dieser Berechnung einem Modul für Maschinenlernen **4** direkt zugeführt haben, basiert die Erfindung darauf, ein Datenkennzeichnungskorrektur-Subsystem (**11**, **12**, **13** und **14**) zu etablieren. Dabei wird zunächst ein Ähnlichkeitsgraph **11** errechnet. In einem nächsten Schritt wird ein probabilistisches Modell in Form eines Conditional Random Field (CRF) **12** erstellt. Dieses Modell speist sich ebenfalls aus Experten-generierten Datenkennzeichnungen **2**.

Anschließend können nach Anwendung des CRF verborgene Datenkennzeichnungen **13** abgeleitet werden. Dies führt zu einer möglichen Korrektur der verborgenen Datenkennzeichnungen **14**. Diese werden dann einem Modul für Maschinenlernen **4** zugeführt, das schließlich ein Klassifizierungsmodell **5** generieren kann.

**Figur 3** zeigt schematisch die Verknüpfung der Datenkennzeichnungen für ungeordnete Zellen. Hier wird eine verborgene Datenkennzeichnung von Zelle A **21** mit einer Experten-generierte Datenkennzeichnung von Zelle A **22,** eine verborgene Datenkennzeichnung von Zelle B **23** mit einer Experten-generierte Datenkennzeichnung von Zelle B **24,** eine verborgene Datenkennzeichnung von Zelle C **25** mit einer Experten-generierte Datenkennzeichnung von Zelle C **26,** eine verborgene Datenkennzeichnung von Zelle D **27** mit einer Experten-generierte Datenkennzeichnung von Zelle D **28,** eine verborgene Datenkennzeichnung von Zelle E **29** mit einer Experten-generierte Datenkennzeichnung von Zelle E **30,** eine verborgene Datenkennzeichnung von Zelle F **31** mit einer Experten-generierte Datenkennzeichnung von Zelle F **32** und eine verborgene Datenkennzeichnung von Zelle G **33** mit einer Experten-generierte Datenkennzeichnung von Zelle G **34** mittels gerichteter Kanten verknüpft **36**. Eine Verknüpfung der verborgenen Datenkennzeichnungen untereinander ist mittels ungerichteter Kanten **35** vorgesehen.

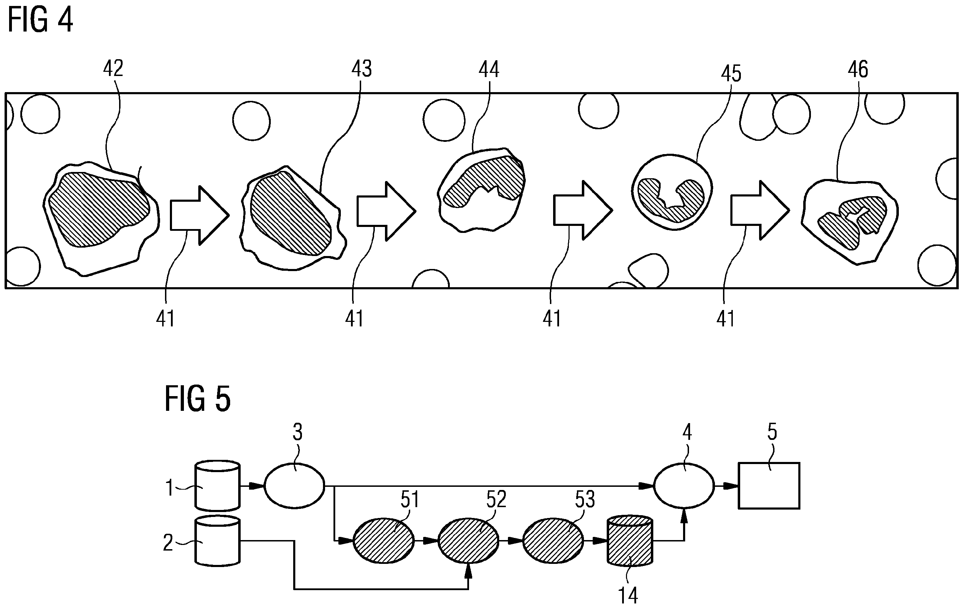

**Figur 4** zeigt eine Transition zwischen verschiedenen Zellentwicklungsstadien **41** am Beispiel von Granulozyten. Von links nach rechts sind die Stadien Myeloblast **42**, Promyelozyt **43**, Myelozyt **44**, Metamyelozyt **45** und stabkerniger neutrophiler segmentkerniger Granulozyt **46** dargestellt.

Die abgebildeten Zellen entsprechen einer geordneten Abfolge und unterscheiden sich von willkürlichen Klassen durch inhärente Ordnungsprinzipien, wie ähnlichen Aufbau, ähnliche Größe, aber Unterschieden in der Morphologie des Zellkerns etc.

**Figur 5** zeigt ein erfindungsgemäßes schematisches Berechnungsverfahren auf Basis von Zellen, welche einer Transition von Zellentwicklungsstadien unterliegen. Dabei wird mit Bilddaten **1** zunächst eine Merkmalsberechnung **3** durchgeführt. Während die bisherigen Verfahren die Ergebnisse dieser Berechnung einem Modul für Maschinenlernen **4** direkt zugeführt haben, basiert die Erfindung darauf, ein Datenkennzeichnungskorrektur-Subsystem (**51, 52, 53** und **14**) zu etablieren. Dabei wird zunächst eine Pseudozeit **51** errechnet. In einem nächsten Schritt wird ein probabilistisches Modell in Form eines Hidden Markov Modells (HMM) **52** erstellt. Dieses Modell speist sich ebenfalls aus Experten-generierten Datenkennzeichnungen **2**.

Anschließend können nach Anwendung des HMM Datenkennzeichnungsgrenzen in der Pseudozeit **53** abgeleitet werden. Dies führt zu einer möglichen Korrektur der verborgenen Datenkennzeichnungen **14**. Diese werden dann einem Modul für Maschinenlernen **4** zugeführt, das schließlich ein Klassifizierungsmodell **5** generieren kann.

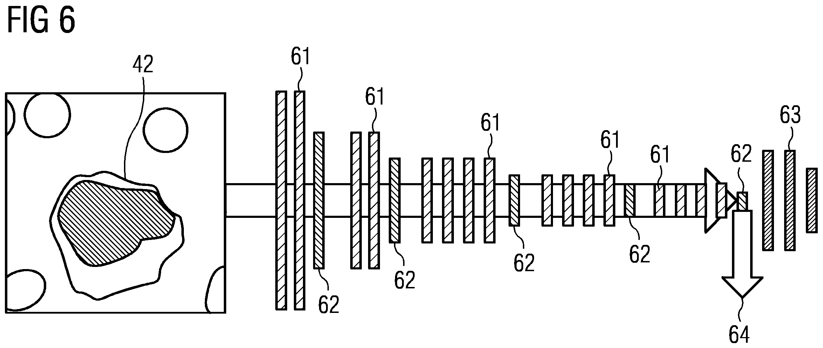

**Figur 6** zeigt ein Deep Convolutional Neural Network (DCNN) für die Berechnung eines Merkmalsraumes für Trainingsdatensätze. Ausgangspunkt ist das Bild eines Myeloblasten **42** als Trainingsdatensatz, dessen Merkmale mittels eines Neuronalen Netzwerkes durch Faltung **61,** Max-Pooling **62** und Verdichtung **63** analysiert werden, um eine Repräsentation im Merkmalsraum **64** zu erhalten.

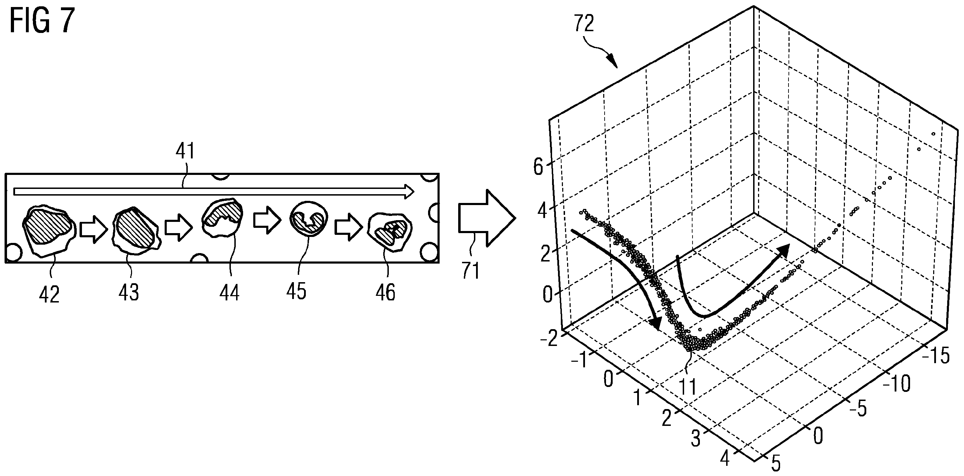

**Figur 7** zeigt die Darstellung von Zellen im Merkmalsraum. Im linken Bildbereich ist die Transition zwischen verschiedenen Zellentwicklungsstadien **41** am Beispiel von Granulozyten zu erkennen. Von links nach rechts sind die Stadien Myeloblast **42**, Promyelozyt **43**, Myelozyt **44**, Metamyelozyt **45** und stabkerniger neutrophiler segmentkerniger Granulozyt **46** dargestellt. Nach einer Transformation **71** wird ein Merkmalsraum **72** erstellt, welcher in Form eines Ähnlichkeitsgraphen **11** repräsentiert sein kann. Die abgeleiteten Informationen zu den Zellen sind dabei in Form des Ähnlichkeitsgraphen **11** angeordnet, wobei der Kurvenverlauf im Verhältnis zum Entwicklungsstadium der Zellen angeordnet ist.

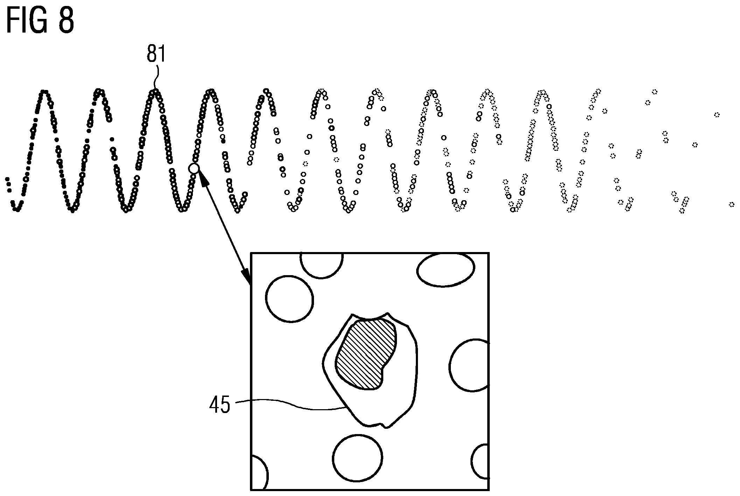

**Figur 8** reflektiert die Reihenfolge von Zellen in der Pseudozeit **81,** wobei eine Verknüpfung zwischen der Pseudozeit und der Repräsentation gem. Bilddaten hergestellt werden kann. Als Beispiel ist die Positionierung eines Metamyelozyten **45** dargestellt.

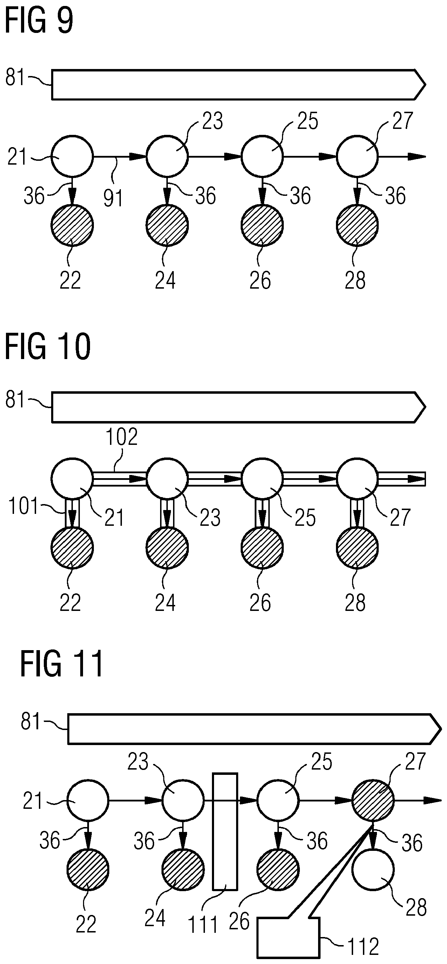

**Figur 9** zeigt die Verknüpfung von verborgenen Datenkennzeichnungen und Experten-generierten Datenkennzeichnungen als Ausgangssituation für die Anwendung eines Hidden Markov Models im Rahmen der Analyse von Zellen die einer Transition zwischen verschiedenen Zellentwicklungsstadien unterliegen, also nicht ungeordnet sind. Diese werden zunächst im Rahmen der errechneten Pseudozeit **81** angeordnet. Anschließend werden die verborgene Datenkennzeichnung von Zelle A **21** mit einer Experten-generierte Datenkennzeichnung von Zelle A **22,** eine verborgene Datenkennzeichnung von Zelle B **23** mit einer Experten-generierte Datenkennzeichnung von Zelle B **24,** eine verborgene Datenkennzeichnung von Zelle C **25** mit einer Experten-generierte Datenkennzeichnung von Zelle C **26,** eine verborgene Datenkennzeichnung von Zelle D **27** mit einer Experten-generierte Datenkennzeichnung von Zelle D **28** verknüpft. Der Abstand zwischen den Zellen im Rahmen des Pseudozeitkonzeptes wird als Distanz zur nächsten Zelle in der Pseudozeit **91** gezeigt. Die verborgenen Datenkennzeichnungen und die Experten-generierten Datenkennzeichnungen sind mittels gerichteter Kanten **36** verknüpft.

**Figur 10** zeigt die Verknüpfung von verborgenen Datenkennzeichnungen und Experten-generierten Datenkennzeichnungen nach Anwendung eines Hidden Markov Models im Rahmen der Analyse von Zellen die einer Transition zwischen verschiedenen Zellentwicklungsstadien unterliegen, also nicht ungeordnet sind. Diese werden zunächst im Rahmen der errechneten Pseudozeit **81** angeordnet. Anschließend werden die verborgene Datenkennzeichnung von Zelle A **21** mit einer Experten-generierte Datenkennzeichnung von Zelle A **22,** eine verborgene Datenkennzeichnung von Zelle B **23** mit einer Experten-generierte Datenkennzeichnung von Zelle B **24,** eine verborgene Datenkennzeichnung von Zelle C **25** mit einer Experten-generierte Datenkennzeichnung von Zelle C **26,** eine verborgene Datenkennzeichnung von Zelle D **27** mit einer Experten-generierte Datenkennzeichnung von Zelle D **28** verknüpft. Hierbei erfolgt eine Codierung der Verknüpfungen durch die Transitionsmatrix des HMM **102,** sowie eine Codierung in Emissionswahrscheinlichkeit des HMM **101.** Die verborgenen Datenkennzeichnungen und die Experten-generierten Datenkennzeichnungen sind mittels gerichteter Kanten **36** verknüpft.

**Figur 11** zeigt Diskrepanzen zwischen den verborgenen Datenkennzeichnungen und den Experten-generierten Datenkennzeichnungen, welche zur Aufdeckung von Fehlbewertungen bzw. Fehlklassifikationen führen können. Zunächst erfolgte eine Anordnung im Rahmen der errechneten Pseudozeit **81.** Nach einer Verknüpfung der verborgene Datenkennzeichnung von Zelle A **21** mit einer Experten-generierte Datenkennzeichnung von Zelle A **22,** der verborgene Datenkennzeichnung von Zelle B **23** mit einer Experten-generierte Datenkennzeichnung von Zelle B **24,** der verborgenen Datenkennzeichnung von Zelle C **25** mit einer Experten-generierte Datenkennzeichnung von Zelle C **26,** und der verborgene Datenkennzeichnung von Zelle D **27** mit einer Experten-generierte Datenkennzeichnung von Zelle D **28** kann zunächst eine geschätzte Klassen- bzw. Entwicklungsstadiengrenze **111** etabliert werden. Die verborgenen Datenkennzeichnungen und die Experten-generierten Datenkennzeichnungen sind dabei mittels gerichteter Kanten **36** verknüpft. Hier zeigt sich jedoch, dass eine Experten-generierte Datenkennzeichnung **28** auf eine andere Klasse verweist, als die damit assoziierte, in der Pseudozeit jüngere verborgene Datenkennzeichnung **27.** Dies deutet auf einen Bewertungsfehler des Experten **112** hin.

Die identifizierte Diskrepanz mit vermutetem Bewertungsfehler zwischen den Datenkennzeichnungen kann anschließend in Form eines Feedbacks an den Experten zurückgespielt werden, sodass eine Neubewertung oder Überprüfung möglich ist. Damit ist unter anderem ein effizientes Assistenzsystem zur Zellklassifizierung umsetzbar.

Bezugszeichen

1    Bilddaten
2    Experten-generierte Datenkennzeichnungen

3   Merkmalsberechnung
4   Maschinenlernen
5   Klassifizierungsmodell

11   Berechnung des Ähnlichkeitsgraph
12   Erstellung eines probabilistischen Modells in Form eines Conditional Random Field (CRF)
13   Ableitung von verborgenen Datenkennzeichnungen
14   Korrektur der verborgenen Datenkennzeichnungen

21   Verborgene Datenkennzeichnung von Zelle A
22   Experten-generierte Datenkennzeichnung von Zelle A
23   Verborgene Datenkennzeichnung von Zelle B
24   Experten-generierte Datenkennzeichnung von Zelle B
25   Verborgene Datenkennzeichnung von Zelle C
26   Experten-generierte Datenkennzeichnung von Zelle C
27   Verborgene Datenkennzeichnung von Zelle D
28   Experten-generierte Datenkennzeichnung von Zelle D
29   Verborgene Datenkennzeichnung von Zelle E
30   Experten-generierte Datenkennzeichnung von Zelle E
31   Verborgene Datenkennzeichnung von Zelle F
32   Experten-generierte Datenkennzeichnung von Zelle F
33   Verborgene Datenkennzeichnung von Zelle G
34   Experten-generierte Datenkennzeichnung von Zelle G
35   Verknüpfung der verborgenen Datenkennzeichen untereinander
36   Verknüpfung der verborgenen Datenkennzeichen und der Experten-generierte Datenkennzeichnungen

41   Transition von Zellentwicklungsstadien
42   Myeloblast
43   Promyelozyt
44   Myelozyt
45   Metamyelozyt
46   stabkerniger neutrophiler segmentkerniger Granulozyt

51   Berechnung der Pseudozeit
52   Erstellung eines probabilistischen Modells in Form eines Hidden Markov Modells (HMM)
53   Ableitung von der Datenkennzeichnungsgrenzen in der Pseudozeit
61   Faltung
62   Max-Pooling
63   Verdichtung
64   Repräsentation im Merkmalsraum

71   Transformation
72   Merkmalsraum
73   Zellen im Rahmen ihrer Entwicklung im Merkmalsraum

81   Zellen in der Pseudozeit

91   Distanz zur nächsten Zelle in der Pseudozeit

101   Codierung in Emissionswahrscheinlichkeit des HMM
102   Codierung durch Transitionsmatrix des HMM
111   geschätzte Klassen- bzw. Entwicklungsstadiengrenze
112   Fehler des Experten

Beispiele

<u>Beispiel 1</u>

Pseudozeit-Inferenz

Die Pseudozeit einer Zelle beschreibt den Entwicklungsfortschritt der Zelle entlang eines dynamischen Prozesses wie der Zelldifferenzierung. Je länger die Pseudozeit einer Zelle ist, desto ausdifferenzierter ist die Zelle. Mithilfe von Pseudozeit-Inferenz-Algorithmen kann man eine pseudotemporale Reihenfolge für alle Zellen in einer Population erstellen. Pseudozeit-Inferenzalgorithmen werden normalerweise auf Einzelzell-Genexpressions-Ähnlichkeitsmessungen angewendet (Haghverdi et al., 2016, Nature Methods, 13(10), 845-848), bei denen benachbarte Zellen eine höhere Expressionsähnlichkeit aufweisen. Diese Algorithmen können auf medizinische Bilder angewendet werden, indem die Pixel eines Zellbildes als Informationen bezüglich dieser Zelle interpretiert werden, ähnlich wie bei Genexpressionsdaten, um eine Reihenfolge der Zellen entlang der Entwicklungslinien zu erhalten. Bisher gibt es eine Vielzahl von Pseudozeit-Inferenzmethoden, die sich in den Anforderungen an vorhandene Vorinformationen, Skalierbarkeit und Art der Topologie unterscheiden (Saelens et al., 2019, Nature Biotechnology, 37,547-554). Die meisten Pseudozeit-Inferenzmethoden bestehen aus zwei Teilen. Der erste Teil ist die Berechnung einer niedrigdimensionalen Darstellung aus den gegebenen Expressionsdaten der Zellen, und der zweite Teil ist die Reihenfolge der Zellen entlang einer abgeleiteten Entwicklungslinie. Hier wurden die Algorithmen SCORPIUS (Cannoodt, 2016, SCORPIUS improves trajectory inference and identifies novel modules in dendritic cell development, bioRxiv:10.1101/079509v2) und STREAM (Chen et al., 2019, Nature Communications, 10, 1, 1903) verwendet. SCORPIUS zeigt eine sehr gute Leistung für lineare Datensätze, während STREAM für Datensätze mit baumartigen Topologien gut geeignet ist. SCORPIUS erhält angesichts der Expressionsprofile der Zellen eine niedrigdimensionale Darstellung unter Verwendung des multi-dimensional scaling (MDS). Als nächstes wendet SCORPIUS k-means clustering an und legt die anfängliche Entwicklungslinie durch Verbinden der Clusterzentren fest. Die endgültige Entwicklungslinie ergibt sich aus einer iterativen Verfeinerung durch den Hauptkurvenalgorithmus. Die Pseudozeit wird berechnet, indem die niedrigdimensionalen Darstellungen auf die Entwicklungslinie projiziert werden. In ähnlicher Weise bestimmt STREAM zuerst relevante Merkmale und führt dann eine Dimensionsreduktion unter Verwendung einer modifizierten lokal linearen Einbettung (modified locally linear embedding (MLLE)) durch. In der neuen Einbettung wird eine Implementierung von elastischen Hauptgraphen (ElPiGraph) (Albergante et al., 2018, Robust and scalable learning of complex datase ttopologies via ElPiGraph, arXiv:1804.07580v2) verwendet, um die Entwicklungslinie und die Verzweigungspunkte abzuleiten. ElPiGraph approximiert Datensätze mit komplexen Topologien, indem es die elastische Energie der Einbettung minimiert und Graphentransformationen anwendet. Die Zellen werden dann entsprechend ihrer Pseudozeit und ihrer zugewiesenen Zweige auf den resultierenden Baum projiziert (siehe auch Chen et al., 2019, Nature Communications, 10, 1, 1903).

<u>Beispiel 2</u>

Hidden Markov-Bäume

Hidden Markov-Bäume werden verwendet, um den Differenzierungsprozess der Zellen zu beschreiben, der nach der Markov-Annahme ein stochastischer Prozess ist (Abkowitz et al., 1996, Nature Medicine, 2, 2, 190-197). Es gibt einen Wurzelzelltyp, und alle anderen Zelltypen entwickeln sich daraus und können auf eine baumartige Topologie abgebildet werden, die ihre jeweiligen Nachkommen widerspiegelt. Unter der Annahme, dass die Topologie des Datensatzes bekannt ist, d.h. dass die Form des Markov-Baumes bekannt ist, gilt:

Definition 1: Ein Baum $\overline{Z1}$ ist ein Markov-Baum, wenn für jedes Blatt der gerichtete Pfad, der die Wurzel und das Blatt verbindet, eine Markov-Kette ist. Ein Hidden Markov-Baum ist eine Erweiterung eines Markov-Baums und wird für Anwendungen verwendet, bei denen die Markov-Eigenschaft nicht gilt oder bei denen die Zustände nur indirekt beobachtet werden können. Das Modell besteht aus beobachteten Variablen und versteckten Variablen, wobei nur die versteckten Variablen der Markov-Eigenschaft folgen. Die gegenwärtig beobachtete Variable hängt vom gegenwärtigen verborgenen Zustand ab, jedoch weder von früheren beobachteten Zuständen noch von früheren verborgenen Zuständen.
Es wird definiert: $\overline{Z1} := (Z1, ..., ZT)$ und $\overline{X1} := (X1, ..., XT)$, für $T \in N$ als Hidden Baum bzw. als beobachteter Baum. Die Wurzeln der Bäume sind Z1 und X1, und beide Bäume haben die gleiche Indexierungsstruktur.

Definition 2: $\overline{X1}$ und $\overline{X1}$ seien zwei Bäume, wobei $\overline{X1}$ der beobachtete Baum und $\overline{X1}$ der Hidden Baum ist. Das Paar $(\overline{X1}, \overline{X1})$ ist ein Hidden Markov-Baum (HMT), wenn

(i) $\overline{X1}$ ein Markov-Baum ist und

(ii) die Verteilung der beobachteten Variablen Xt nur von der versteckten Variablen Zt für alle $t \in \{1,\ldots,T\}$ abhängt.

Für die Anwendung auf Zellbild-Datenkennzeichnungen entspricht die Variable Xt der fehlerhaften (beobachteten) Experten-Datenkennzeichnung, und Zt repräsentiert die wahre (nicht beobachtbare) Datenkennzeichnungen des Bildes, das sich von der Experten-Datenkennzeichnungen unterscheiden kann. Die Folge von Bildern wird durch Erhöhen der Pseudozeit sortiert, die zuvor durch einen geeigneten Pseudozeit-Inferenzalgorithmus berechnet wurde. Es sei K die Anzahl der Zelltypen und T die Anzahl der Bilder im Datensatz.

Definition 3: Der Hidden Markov-Baum $(\overline{X1}, \overline{X1})$ wird durch die Parameter $\pi \in [0, 1]^K$, $A^{(t)} \in [0, 1]^{K \times K}$ und $B \in [0, 1]^{K \times K}$ bestimmt. Für $2 \le t \le T$, $1 \le k$, $\tilde{k} \le K$ wird definiert

$$\boldsymbol{\pi}_k := \mathbb{P}(Z_1 = k),$$

$$\boldsymbol{A}_{kl}^{(t)} := \mathbb{P}(Z_t = l \mid Z_{\mathfrak{p}(t)} = k),$$

$$\boldsymbol{B}_{k\tilde{k}} := \mathbb{P}(X_t = \tilde{k} \mid Z_t = k),$$

wobei p(t) das Elternteil des Knotens t bezeichnet.

n wird als Startwahrscheinlichkeit bezeichnet, $A^{(t)}$ als die Übergangsmatrix am Knoten t und B als die Emissionsmatrix. Wenn die Übergangsmatrix $A^{(t)}$ unabhängig von t ist, wird das Modell als homogen bezeichnet; andernfalls wird das Modell als inhomogen bezeichnet. Die Übergangsmatrix $A^{(t)}$ beschreibt die Wahrscheinlichkeit, im gegenwärtigen Zelltyp zu bleiben oder zu einem untergeordneten Zelltyp zu wechseln. Die Emissionsmatrix B stellt das Experten-Datenkennzeichnungs-Fehlermodell dar, wobei $B_{kn}$ die Wahrscheinlichkeit ist, dass der Experte die Datenkennzeichnung $\tilde{k}$ vorhersagt, wenn der wahre Zelltyp der Zelle im Bild k ist.

Ein Hidden-Markov-Modell (HMM) ist ein Sonderfall eines HMT, bei dem die zugrunde liegende Topologie eine Kette ist.

Zeitabhängige Übergangsmatrizen

Es wurden die folgenden Informationen verwendet, um die parametrischen Übergangsmatrizen einzurichten. Die Topologie des Datensatzes ist bekannt und nach der Markov-Annahme der Blutzelldifferenzierung (Abkowitz et al., 1996, Nature Medicine, 2, 2, 190-197) ist es nur möglich, dass eine Zelle derselbe Zelltyp bleibt oder in einen der untergeordneten Zelltypen übergeht. Es gibt keine Möglichkeit, einen Zelltyp zu überspringen oder zu einem vorherigen Zelltyp zurückzukehren. Sobald eine der Endstufen erreicht ist, gibt es keine Übergänge mehr. Standardmäßige homogene HMMs / HMTs basieren auf der Annahme, dass der Übergang zwischen Zuständen unabhängig von t ist, was Zellen entsprechen würde, die gleichmäßig über die Entwicklungslinie abgetastet werden. In der Praxis stammen diese Stichproben (d.h. die gekennzeichneten Zellen) jedoch von beliebigen Punkten auf der Entwicklungslinie, was sich in einer großen Variation der Pseudozeit-Differenz zwischen benachbarten Zellen widerspiegelt. Dieser Unterschied wirkt sich direkt auf die Wahrscheinlichkeit aus, dass eine Zelle zu einem anderen Zelltyp übergeht: je größer der Pseudozeit-Unterschied zwischen zwei Zellen ist, desto größer ist die Wahrscheinlichkeit für einen Übergang (und desto geringer ist die Wahrscheinlichkeit, dass die Zelle denselben Zelltyp beibehält). Folglich sollten die Einträge der Übergangsmatrix am Knoten t nicht nur vom Zelltyp der vorherigen Zelle abhängen, sondern auch von der Zeitdifferenz zwischen der gegenwärtigen Zelle und der vorherigen Zelle. Um die Abhängigkeit der Übergangsmatrix von der Pseudozeit zu modellieren, wurden die Algorithmen für HMMs und HMTs auf den inhomogenen Fall erweitert und es wurden geeignete parametrische Übergangsmatrizen abgeleitet.

Es gelten die Definitionen:

yt $\in$ R≥0 als Pseudozeit-Differenz zwischen Zelle t - 1 und Zelle t, nachdem sie durch Erhöhen der Pseudozeit geordnet wurden. Um vernünftige Einträge für die Übergangsmatrizen zu finden, werden die Übergangswahrscheinlichkeiten am Knoten t definiert als:

$$\boldsymbol{A}_{kl}^{(t)} = \mathbb{P}(Z_t = l | Z_{t-1} = k, y_t)$$

$$= \frac{\mathbb{P}(Z_t = l, Z_{t-1} = k, y_t)}{\mathbb{P}(Z_{t-1} = k, y_t)}$$

$$= \frac{\mathbb{P}(Z_{t-1} = k)\mathbb{P}(Z_t = l | Z_{t-1} = k)\mathbb{P}(y_t | Z_t = l, Z_{t-1} = k)}{\mathbb{P}(Z_{t-1} = k)\mathbb{P}(y_t | Z_{t-1} = k)}$$

$$= \frac{\mathbb{P}(Z_t = l | Z_{t-1} = k)\mathbb{P}(y_t | Z_t = l, Z_{t-1} = k)}{\mathbb{P}(y_t | Z_{t-1} = k)}.$$

Dabei gilt P ($Z_t$ = l | $Z_{t-1}$ = k) =: $p_{kl}$ ∈ [0, 1] als Übergangswahrscheinlichkeit vom Zelltyp k zum Zelltyp l. Es sei $p_{kl}$ eine von t unabhängige Konstante mit der Bedingung $\sum_{l=1}^{k} pkl = 1$ für alle k.

Für die Wahrscheinlichkeit P ($y_t$ | $Z_t$ = l, $Z_{t-1}$ = k) ist bekannt, dass der Träger von $y_t$ [0, ∞) ist. Da es keine weiteren Informationen über die Verteilung der Pseudozeit-Differenz gibt, wird die Entropie maximierende Wahrscheinlichkeitsverteilung verwendet. Die am wenigsten informative Verteilung für eine Zufallsvariable mit Träger [0, ∞) und Mittelwert 1 / λ ist die Exponentialverteilung mit der Rate λ. Die Rate λ sei abhängig von den Zelltypen k und l.

Dann hat für jeden möglichen Übergang im Zelllinienbaum der Eintrag in der Übergangsmatrix nach der Normalisierung die Form

$$\boldsymbol{A}_{kl}^{(t)} = \frac{p_{kl} \cdot \lambda_{kl} \exp(-\lambda_{kl} \cdot y_t)}{\sum_{i=1}^{K} p_{ki} \cdot \lambda_{ki} \exp(-\lambda_{ki} \cdot y_t)}$$

für $p_{kl}$ ∈ [0, 1] und $\lambda_{kl}$ > 0.

Die Parameter in dieser Formel werden unter Verwendung des verallgemeinerten EM-Algorithmus (Neal und Hinton, 1998, A view of the EM algorithm that justifies incremental, sparse, and other variantsLearning, in Graphical Models, 355-368) gelernt, da die entsprechende Zielfunktion unlösbar ist.

Der verallgemeinerte Viterbi-Algorithmus (Durand et al., 2004, IEEE Transactions on Signal Processing, 52(9), 2551-2560) berechnet dann die wahrscheinlichsten verborgenen Variablen arg max $_{z1: T}$ P (Z1: T | X1: T) .

Beispiel 3

Der Algorithmus TIMELY

TIMELY kombiniert Pseudozeit-Inferenzmethoden mit inhomogenen HMTs. Der Pseudozeit-Inferenzalgorithmus legt eine intrinsische Reihenfolge der Zellen basierend auf der Morphologie fest, und das HMT findet dann inkonsistente Datenkennzeichnungen und schlägt korrekte Datenkennzeichnungen der Zellen vor, die den wahren Zelltypen entsprechen. Die Eingabe von TIMELY besteht aus einer Reihe von Bildern zusammen mit fehlerhaften Experten-Datenkennzeichnungen. Zunächst wird ein Netzwerk (convolutional network) verwendet, um aussagekräftige Merkmalsdarstellungen der Zellbilder zu lernen, die mit der Morphologie der Zellen übereinstimmen. Das faltende Netzwerk besteht aus drei Faltungsschichten mit jeweils 32 Filtern, wobei die Filtergröße 3 × 3 beträgt. Nach jeder Faltungsschicht gibt es eine Max-Pooling-Schicht mit einer Pooling-Größe von 2 × 2. Einem Engpass von 50 Einheiten, der die resultierenden Merkmalsvektoren bereitstellt, folgen zwei dichte Schichten mit jeweils 30 verborgenen Einheiten und eine Ausgangsschicht.

Alternativ wurden auch nicht-überwachte Methoden wie Autoencoder untersucht, um Merkmals-Darstellungen der Bilder zu lernen, damit das Training nicht durch fehlerhafte Datenkennzeichen beeinträchtigt wird. Dies ergab qualitativ ähnliche Ergebnisse.

Als nächstes wurde ein geeignetes Pseudozeit-Inferenzverfahren angewendet, um die Pseudozeit zu berechnen. Die Zellen wurden nach zunehmender Pseudozeit geordnet. Es wurde, abhängig von der Topologie der Daten, entweder SCORPIUS oder STREAM verwendet. Die sortierten Experten-Datenkennzeichnungen dienten als beobachtete Informationen in der HMT, und die versteckten Datenkennzeichnungen sind die wahren zu bestimmenden Zelltypen. Die Hintergrundinformationen über den Datensatz können verwendet werden, um die Startwahrscheinlichkeiten $\Pi$ und die Emissionsmatrix B zu fixieren, während die Parameter der Übergangsmatrizen vom verallgemeinerten EM-Algorithmus gelernt werden. Durch den verallgemeinerten Viterbi-Algorithmus wurden die wahrscheinlichsten wahren Datenkennzeichnungen und die geschätzten Zelltypgrenzen gefunden, die aufgrund der Markov-Annahme eindeutig sind (Abkowitz et al., 1996, Nature Medicine, 2, 2, 190-197).

Inkonsistenzen zwischen den echten Datenkennzeichnungen und den Experten-Datenkennzeichnungen sind potenzielle Fehler des Experten. Hämatologen können die betroffenen Bilder erneut untersuchen und gegebenenfalls die Datenkennzeichnungen der Zellen korrigieren. Die Methode ist in Algorithmus 1 (siehe unten) zusammengefasst. TIMELY wurde in Python implementiert, und die Bibliothek SciPy wurde zur Maximierung der Zielfunktion im verallgemeinerten EM-Algorithmus verwendet.

Algorithmus 1 TIMELY:

Eingabe: Bilder und fehlerhafte Experten-Datenkennzeichnungen

Ausgabe: Bilder mit inkonsistenten Datenkennzeichnungen und vorgeschlagenen Datenkennzeichnungen

1: Verwenden von Hintergrundinformationen zum Datensatz, um die Topologie des HMT, die Startwahrscheinlichkeiten $\Pi$ und die Emissionsmatrix B zu definieren.
2: Lernen der Merkmalsdarstellungen der Bilder unter Verwendung eines neuronalen Netzwerkes.
3: Wählen eines geeigneten Pseudozeit-Inferenzalgorithmus und Berechnen der Pseudozeiten anhand der Merkmalsvektoren.
4: Sortieren der entsprechenden Experten-Datenkennzeichnungen nach aufsteigender Pseudozeit.
5: Einrichten eines HMT, bei dem die sortierten Experten-Datenkennzeichnungen die beobachteten Informationen sind.
6: Lernen der Parameter in den Übergangsmatrizen A (t) unter Verwendung des verallgemeinerten EM-Algorithmus. 7: Anwenden des verallgemeinerten Viterbi-Algorithmus, um die wahrscheinlichsten wahren Datenkennzeichnungen abzuleiten.
8: Identifizieren von Bildern mit inkonsistenten Datenkennzeichnungen, indem die tatsächlichen Datenkennzeichnungen mit den Experten-Datenkennzeichnungen verglichen werden.

Beispiel 4

Basisverfahren

TIMELY wurde mit drei Basisverfahren verglichen. Wie oben erläutert, sind die meisten Algorithmen entweder robust gegenüber fehlerhaften Datenkennzeichnungen, finden und entfernen fehlerhafte Datenkennzeichnungen oder modellieren fehlerhafte Datenkennzeichnungen explizit, schlagen jedoch keine neuen Datenkennzeichnungen vor.

Die Algorithmen k-nearest neighbors (k-NN) und k-nearest centroid neighbors (k-NCN) (Sanchez et al., 1997, Pattern Recognition Letters, 18, 11-13, 1179-1186) finden Nachbarn für jede Instanz eines gegebenen Abstandsmaßes. Ein häufig verwendetes Abstandsmaß für k-NN ist der euklidische Abstand, während für k-NCN Instanzen zur Menge der nächsten Nachbarn hinzugefügt wurden, für die der Schwerpunkt der neuen Menge der betrachteten Instanz am nächsten liegt. Die Datenkennzeichnung der betrachteten Instanz wird dann über eine Mehrheitsauswahl erhalten. Wenn die Mehrheitsauswahl eine andere Datenkennzeichnung als die ursprüngliche Datenkennzeichnung der Instanz ergibt oder wenn ein Gleichstand besteht, ist die Instanz möglicherweise falsch gekennzeichnet.

Um dieses Verfahren mit anderen Verfahren zu vergleichen, die ebenfalls Korrekturen vorschlagen, wurden diese beiden Verfahren um eine allgemeine Bearbeitung (Koplowitz und Brown, 1981, Pattern Recognition, 13, 3,251-255) erweitert, d.h. es wurden die Zahlen k und k' mit $(k + 1) / 2 \leq k' \leq k$ für k-NN und k-NCN ausgewählt. Für jede Instanz, wenn mindestens k' nächste Nachbarn von einem anderen Zelltyp vorhanden sind, wird der Zelltyp der Instanz in jenen Typ geändert. Anders als in Koplowitz und Brown, 1981 wurden keine Samples gelöscht. Für beide Methoden wurden k = 3 und k' = 2 gewählt, die in der Literatur übliche Werte sind (Saez et al., 2015 Journal of Medical Informatics & Technologies, 24, pp. 123-130).

TIMELY wurde auch mit cleanlab verglichen (Northcutt et al., 2019, Confident learning: estimating uncertainty in dataset labels, arXiv:1911.00068v1), das auf konfidentem Lernen basiert (Northcutt et al., 2017, Learning with confident examples: rank pruning for robust classification with noisy labels, in Proceedings of the 33rd Conference on Uncertainty in Artificial Intelligence. AUAI Press) und Datenkennzeichnungsfehler feststellt. Es schätzt die Fehlerraten, indem es die gemeinsame Verteilung zwischen fehlerhaften und unverfälschten Datenkennzeichnungen berechnet und dann inkonsistente Proben entfernt.

Beispiel 5

Simulationsdaten

Da Experten-Datenkennzeichnungen aus realen Datensätzen häufig fehlerhaft gekennzeichnet sind, kennt man die Referenz-Datenkennzeichnungen der Bilder nicht. Um den hierin beschriebenen Algorithmus mit anderen Methoden zum Auffinden inkonsistenter Datenkennzeichnungen zu vergleichen, wurden drei Datensätze mit unterschiedlichen Fehlerraten simuliert, die die Einstellung der Zelldifferenzierung nachahmen. Jeder Datensatz besteht aus 250 Proben aus fünf Zelltypen, wobei die zugrunde liegende Topologie eine Kette ist. Der Prozess der Simulation der Datensätze ist folgender:

1. Es gilt $X \in R^{2 \times 250}$, wobei X normalverteilt ist.
2. Die Spalten von X werden durch Erhöhen von $X_{1j}$, $1 \leq j \leq 250$ sortiert.
3. Die entsprechenden Referenzbezeichnungen werden definiert als $Y \in R^{250}$, wobei die Einträge $Y_{50 (i-1)+1: 50i}$ i sind für $i \in \{1 ... 5\}$.
4. P wird auf das Projekt X auf einen höherdimensionalen Raum abgebildet mit $\tilde{X} = PX \in R^{k \times 250}$. Es wird k = 50 gewählt, um mit den realen Datensätzen übereinzustimmen.
5. Zu den Referenz-Datenkennzeichnungen Y wird die Fehlerrate $l \in \{10, 20, 30\}$ hinzugefügt, indem 1% der Einträge in Y zufällig in verschiedene Kennzeichnungen geändert werden. Die Schritte 1 bis 4 werden für jede Fehlerrate wiederholt.

Die Idee ist, dass die Abtastwerte eine niedrigdimensionale Ordnung haben, die der pseudotemporalen Ordnung entspricht, die durch Reduktion der Dimensionen der höherdimensionalen Merkmalsvektoren erhalten werden kann.

Beispiel 6

Simulationsergebnisse

Die Ergebnisse des Vergleichs sind in Tabelle 1 gezeigt. Die Verfahren k-NN + edit und k-NCN + edit modifizieren die Datenkennzeichnungen während der Anwendung, während k-NN, k-NCN und cleanlab nur mögliche Datenkennzeichnungsfehler finden. TIMELY findet Datenkennzeichnungsfehler und schlägt neue Kennzeichnungen vor, ohne sie direkt zu ändern.

Die vorgeschlagenen Datenkennzeichnungen werden mit den Referenzdatenkennzeichnungen verglichen, um die Genauigkeit zu berechnen. Die ausgewählten Elemente sind die Instanzen, die der Algorithmus als Datenkennzeichnungsfehler markiert hat. Während TIMELY Fehler in einer Größenordnung findet, die dem Fehlerrate ähnlich ist, finden andere Verfahren meist zu viele Fehler, ohne den Recall zu erhöhen. Nur in einem Fall hat k-NCN einen höheren Recall als TIMELY. Das erfindungsgemäße Verfahren hat in allen anderen Fällen die höchste Genauigkeit, Präzision und Recallquote und das höchste F1-Gütemaß. Das Editieren in k-NN und k-NCN verbessert häufig das F1-Gütemaß im Vergleich zu den Versionen ohne Editierung. Die Editierung von Datenkennzeichnungen während der Anwendung beeinflusst jedoch die Klassifizierung nachfolgender Proben, so dass die Genauigkeit abnimmt, wenn zu viele falsch Positive vorliegen.

Tabelle 1: Vergleich mit Basisverfahren für Simulationsdaten. TIMELY übertrifft alle Basisverfahren hinsichtlich der genauen Identifizierung und Korrektur von fehlerhaften Datenkennzeichnungen

| Fehlerrate | Metrik | TIMELY | k-NN | k-NN+ edit | k-NCN | k-NCN+ edit | cleanlab |
|---|---|---|---|---|---|---|---|
| 10 | Fehlerfreiheit | 0.984 | - | 0.920 | - | 0.944 | - |

(fortgesetzt)

| Fehlerrate | Metrik | TIMELY | k-NN | k-NN+ edit | k-NCN | k-NCN+ edit | cleanl ab |
|---|---|---|---|---|---|---|---|
| | Ausgewählte Elemente | 0.108 | 0.164 | 0.152 | 0.152 | 0.144 | 0.112 |
| | Genauigkeit | 0.889 | 0.561 | 0.579 | 0.658 | 0.667 | 0.679 |
| | Recall | 0.960 | 0.920 | 0.880 | 1.000 | 0.960 | 0.760 |
| | F1-Gütemaß | 0.923 | 0.697 | 0.698 | 0.794 | 0.787 | 0.717 |
| 20 | Fehlerfreiheit | 0.992 | - | 0.932 | - | 0.820 | - |
| | Ausgewählte Elemente | 0.192 | 0.292 | 0.236 | 0.324 | 0.284 | 0.256 |
| | Genauigkeit | 1.000 | 0.658 | 0.797 | 0.556 | 0.634 | 0.625 |
| | Recall | 0.960 | 0.960 | 0.940 | 0.900 | 0.900 | 0.800 |
| | F1-Gütemaß | 0.980 | 0.781 | 0.863 | 0.687 | 0.744 | 0.702 |
| 30 | Fehlerfreiheit | 0.972 | - | 0.792 | - | 0.712 | - |
| | Ausgewählte Elemente | 0.300 | 0.416 | 0.340 | 0.484 | 0.404 | 0.272 |
| | Genauigkeit | 0.987 | 0.673 | 0.706 | 0.537 | 0.604 | 0.809 |
| | Recall | 0.987 | 0.933 | 0.800 | 0.867 | 0.813 | 0.733 |
| | F1-Gütemaß | 0.987 | 0.782 | 0.750 | 0.663 | 0.693 | 0.769 |

Beispiel 7

Anwendung auf reale Daten

TIMELY wurde auf zwei Bilddatensätze gefärbter Leukozyten angewendet. Alle Bilder wurden mit einem Digitalmikroskop (Cellavision, Siemens Healthineers AG) erzeugt und von einem Experten gekennzeichnet. Aufgrund der oben beschriebenen Herausforderungen bei der manuellen Kennzeichnung sind die Datenkennzeichnungen fehlerhaft bzw. teilweise falsch. Zur Vorbereitung der Bilder wurde ein dünner Blutfilm auf einen Objektträger aufgebracht und gefärbt. Ein digitales Mikroskop lokalisierte dann die Blutzellen und erstellte entsprechende Bilder. Die Datensätze enthielten Bilder von mehreren Patienten. TIMELY wurde auf den gesamten Datensatz angewendet, um zunächst die Reihenfolge der Bilder zu bestimmen. Dann schlug es eine Datenkennzeichnung für jedes Bild vor. Für einen neuen Patienten können Bilder aus demselben Entwicklungsbaum auf den bereits berechneten Baum abgebildet werden, und konsistente Datenkennzeichnungen können direkt vom Baum abgelesen werden, indem die bereits berechneten Übergangsgrenzen verwendet werden.

Zell-Linien

Datensätze

Der erste Datensatz bestand aus 1000 Zellbildern, die fünf Zelltypen der Entwicklungslinie Granulopoese enthielten. Die Topologie war eine lineare Kette. Es gab 200 Bilder, die von einem Experten als zu jedem der Zelltypen Promyelozyten (PMY), Myelozyten (MY), Metamyelozyten (MMY), stabkernige Neutrophile (BNE) und segmentierte Neutrophile (SNE) gehörend gekennzeichnet wurden.

Parameter in HMM

Vorhandenes Hintergrundwissen über den Datensatz wurde verwendet, um die Startwahrscheinlichkeiten $\Pi$ und die Emissionsmatrix B festzulegen. Der Datensatz hat fünf Zelltypen und ist bekannt, dass der Wurzeltyp im Entwicklungsprozess PMY ist. Es konnten also die Startwahrscheinlichkeiten folgendermaßen festgelegt werden:

$$\Pi := (0,9 \quad 0,025 \quad 0,025 \quad 0,025 \quad 0,025)^T$$

Die erste Zelle sollte sich mit hoher Wahrscheinlichkeit im ersten Zelltyp und in den anderen Zelltypen mit geringer Wahrscheinlichkeit befinden.

Die konstante Emissionsmatrix B basiert auf Schätzungen eines Experten, der die Wahrscheinlichkeit von Datenkennzeichnungsfehlern realistisch einschätzen kann. Die Emissionsmatrix für den ersten Datensatz sieht wie folgt aus:

|       | PMY   | MY    | MMY   | BNE   | SNE   |
|-------|-------|-------|-------|-------|-------|
| PMY   | 0.7   | 0.25  | 0.04  | 0.005 | 0.005 |
| MY    | 0.23  | 0.52  | 0.24  | 0.005 | 0.005 |
| MMY   | 0.03  | 0.17  | 0.75  | 0.045 | 0.005 |
| BNE   | 0.005 | 0.005 | 0.03  | 0.82  | 0.14  |
| SNE   | 0.005 | 0.005 | 0.005 | 0.065 | 0.92  |

Die ausdifferenzierteren Zelltypen stabkernige Neutrophile und segmentierte Neutrophile sind für den Menschen ziemlich leicht zu unterscheiden, während die ersten drei Zelltypen, insbesondere Myelozyten, schwieriger zu kennzeichnen sind.

Pseudozeit-Inferenz

Es wurde der Algorithmus SCORPIUS verwendet, um die Pseudozeiten zu berechnen. Bevor SCORPIUS angewendet wurde, wurden Diffusionskarten (Coifman und Lafon, 2006, Applied and Computational Harmonic Analysis, 21(1), 5-30) zur Dimensionsverminderung verwendet. Anschließend leitete SCORPIUS die Entwicklungslinie direkt ab, ohne MDS durchzuführen.

Visualisierungswerkzeug

Nach der Parameteroptimierung fand das HMM eindeutige Übergangsgrenzen zwischen den Zelltypen. Es wurde ein Visualisierungswerkzeug zum Anzeigen der Bilder bereitgestellt (siehe auch Fig. 8). Die Bilder sind nach der abgeleiteten Pseudozeit geordnet. Jedes Bild entspricht einem Punkt, der gemäß der Expertendatenkennzeichnung hervorgehoben ist. Die vom HMM abgeleiteten Übergangsgrenzen wurden integriert, und die Zwischenräume zwischen den Grenzen wurden gemäß den vorgeschlagenen Zelltypen gekennzeichnet. Inkonsistente Klassifizierungen konnten durch nicht übereinstimmende Kennzeichnungen identifiziert werden. Der Experte kann auf jeden Punkt klicken, um das entsprechende Bild anzuzeigen, und durch Klicken auf die Pfeile zu benachbarten Zellen navigieren. Dadurch erhält er eine Vorstellung davon, warum eine bestimmte Zelle mit einer inkonsistenten Datenkennzeichnung markiert wurde.

Inkonsistente Datenkennzeichnungen

Die Anzahl der konsistenten Datenkennzeichnungen, bei denen die verborgenen Datenkennzeichnungen und die Experten-Datenkennzeichnungen übereinstimmten, betrug laut HMM 72%. Dies bedeutet, dass 280 Bilder mit möglicherweise falschen Datenkennzeichnungen vorhanden sind. Gemäß einer Konfusionsmatrix konnte gezeigt werden, dass die Konsistenz für Myelozyten und Metamyelozyten besonders gering ist. Insgesamt ähnelte die Tendenz der Werte der Einschätzung des Experten zur Emissionsmatrix, wie oben gezeigt.

Experimente zeigten, dass die Ergebnisse in Bezug auf die Emissionsmatrix ziemlich robust sind, so dass kleine Änderungen der Schätzungen die Ergebnisse nicht wesentlich beeinflussten.

Die 280 inkonsistenten Bilder wurden einem Experten zur Neuklassifizierung übermittelt. Für 128 dieser Bilder (45,7%) bestätigte der Experte die vorherigen Datenkennzeichnungen. Für die verbleibenden 152 Zellen hat der Experte sie entweder in die vom HMM vorgeschlagenen Zelltypen umbenannt, oder er konnte keine Datenkennzeichnung mit hohem Vertrauen vergeben, was bedeutet, dass bis zu 54,3% der inkonsistenten Bilder möglicherweise falsche Datenkennzeichnungen aufweisen könnten. Die meisten Neuklassifizierungen betrafen die ersten drei Zelltypen in der Entwicklungslinie, bei denen Änderungen in der Morphologie sehr subtil sein können.

## Zell-Linien Bäume

### Datensatz

Der zweite Datensatz bestand aus 1821 Zellbildern aus zehn Klassen, die Teil eines Entwicklungsprozesses mit Verzweigungspunkten sind. Es gab 200 Bilder, die von einem Experten als zu jedem der Zelltypen Promyelozyten (PMY), Myelozyten (MY), Metamyelozyten (MMY), stabkernige Neutrophile (BNE), segmentierte Neutrophile (SNE), Blasten (BL), Basophile (BA), Eosinophile (EO) und Lymphozyten (LY) gehörend gekennzeichnet wurden. Für die letzte Klasse der Plasmazellen (PC) gab es nur 21 Bilder. Eosinophile und Basophile haben als Vorläufer ebenfalls Myelozyten, Metamyelozyten und stabkernige Neutrophile. Diese zeigen jedoch unterschiedliches Färbeverhalten, als die Vorläufer der segmentierten Neutrophilen. Da diese Zelltypen im Blut ziemlich selten sind, wurden sie nicht in den Datensatz aufgenommen.

### Parameter im HMT

Die Wurzelzelle sollte ein Blast sein, damit der Eintrag für den Blasten in Π sehr hoch ist. Die konstante Emissionsmatrix B basiert wiederum auf Gesprächen mit einem Experten und ist eine konsistente Erweiterung der oben gezeigten Emissionsmatrix. Für die fünf zusätzlichen Zelltypen sollte es nicht zu schwierig sein, sie von den Zelltypen des ersten Datensatzes zu unterscheiden, da sie Teil verschiedener Entwicklungslinien sind. Nur die Blasten haben einige Ähnlichkeit mit den Promyelozyten, die Nachkommen der Blasten sind. Die segmentierten Neutrophilen, Basophilen und Eosinophilen im Endstadium sollten für Experten leicht zu klassifizieren sein.

### Pseudozeit-Inferenz

Wir verwendeten den Algorithmus STREAM, um einen vernünftigen Baum für den Datensatz abzuleiten. Zwei der drei möglichen Verzweigungspunkte stimmten mit den Verzweigungspunkten aus dem Zelllinienbaum überein. Der letzte Verzweigungspunkt, an dem sich die Eosinophilen von den Metamyelozyten abzweigen, ist allerdings unterschiedlich. Im Allgemeinen sind die Eosinophilen nach der Verminderung der Dimension von den anderen Zelltypen im Merkmalsraum weit entfernt. Der Verbindungspunkt zum verbleibenden Baum könnte möglicherweise nicht richtig sein. Ein weiterer Grund könnte darin bestehen, dass die Vorläuferzellen der segmentierten Neutrophilen und der Eosinophilen gleich aussehen. Eosinophile haben die gleichen Vorläufer-Stadien wie die Neutrophilen, die nur in einer anderen Farbe gefärbt sind. Der Algorithmus könnte die Metamyelozyten auch als ein vorheriges Entwicklungsstadium der Eosinophilen identifizieren. Der Bereich der Pseudozeiten ist allerdings für alle Zelltypen noch plausibel.

### Inkonsistente Beschriftungen

Der Prozentsatz konsistenter Beschriftungen gemäß HMT beträgt 69%, was bedeutet, dass 564 Bilder mit möglicherweise falschen Beschriftungen vorhanden sind. Blasten und Promyelozyten scheinen häufig verwechselt zu werden, während Basophile und Eosinophile eine hohe Übereinstimmung zwischen verborgenen Datenkennzeichnungen und Experten-Datenkennzeichnungen aufweisen, vermutlich aufgrund ihrer unterschiedlichen Anfärbung. Die Übereinstimmung bei Lymphozyten ist ebenfalls sehr hoch, da Zellen aus verschiedenen Entwicklungslinien normalerweise leichter zu unterscheiden sind.

Die 564 inkonsistenten Bilder wurden einem Experten zur Neuklassifizierung übermittelt. Der Experte bestätigte für 341 Bilder seine vorherigen Datenkennzeichnungen, so dass bis zu 40,1% der inkonsistenten Bilder möglicherweise falsche Datenkennzeichnungen aufweisen. Die meisten Umklassifizierungen betrafen Promyelozyten, Myelozyten und Metamyelozyten, welche die ersten drei Zelltypen in der Granulopoese darstellen. Die Zellen wurden meist als Vorläufer des von den Experten bestimmten Zelltyps klassifiziert.

### Zusammenfassung der Ergebnisse

Das erfindungsgemäße Verfahren TIMELY wurde eingeführt, um einen innovativen und effizienten, auf den Menschen ausgerichteten Ansatz zur Erhöhung der Datenkennzeichnungskonsistenz in der medizinischen Bildgebung für die Zelltypklassifizierung zu erhalten.

TIMELY verwendet als Eingabe Zell-mikroskopische Bilder zusammen mit fehlerhaften Experten-generierten Datenkennzeichnungen, identifiziert inkonsistente Datenkennzeichnungen und schlägt alternative, konsistente Datenkennzeichnungen vor, die auf einem zweistufigen Verfahren basieren.

Im ersten Schritt stellt TIMELY mithilfe eines Pseudozeit-Inferenzalgorithmus eine intrinsische Reihenfolge zwischen Zellen her. Im zweiten Schritt erstellt TIMELY ein Markov-Modell, welches auf den geordneten Zellen und ihren fehlerhaften Beschriftungen basiert. Abhängig von der Komplexität der Topologie des Datensatzes wird ein HMM oder ein HMT verwendet. Pseudozeit-Schätzungen werden mit interpretierbaren HMTs kombiniert, um ein System zu etablieren, das beispielsweise einem annotierenden Hämatologen oder Histologen hilft, konsistentere Zellklassifizierungen zu generieren. Indem die Zellen nach der Pseudozeit geordnet werden, kann von dem annotierenden Hämatologen oder Histologen jede Zelle in der Nachbarschaft von Zellen mit einer ähnlichen Morphologie betrachtet werden. Er wird somit dabei unterstützt, konsistente Entscheidungen zu treffen. Darüber hinaus wird Domänenwissen transparent und explizit in Form von Differenzierungshierarchien, Startwahrscheinlichkeiten (siehe oben) und einer von Experten gesteuerten Emissionsmatrix (siehe oben) codiert, die frühere Erfahrungen mit der Wahrscheinlichkeit von Datenkennzeichnungsfehlern widerspiegeln.

Zusammengenommen ermöglicht dies bspw. einem Hämatologen oder Histologen, ein intuitives Verständnis dafür zu entwickeln, warum bestimmte Zellen als inkonsistent gekennzeichnet vorgeschlagen werden, und hilft bei einer leichteren Übernahme in die Praxis.

Das manuelle Markieren von Zellen ist ebenfalls ein zeitaufwändiger Prozess, und das erfindungsgemäße Verfahren kann angewendet werden, um die Zeit zu reduzieren, die Experten für diese Aufgabe benötigen.

Sobald die Parameter eines HMT optimiert sind, können neue Bilder aus demselben Entwicklungsbaum auf den bereits berechneten Baum abgebildet werden, und konsistente Datenkennzeichnungen können direkt vom Baum abgelesen werden, indem die bereits berechneten Übergangsgrenzen verwendet werden.

Ein zusätzlicher, beispielhafter Anwendungsfall von TIMELY ist die Anwendung auf automatisch generierte Datenkennzeichnungen, da diese häufig fehlerhaft sind. Darüber hinaus enthält der Klassifizierungsalgorithmus nicht alle möglichen Zelltypen. Diese Datenkennzeichnungen würden dann als beobachtete Information des HMT dienen, und nur die inkonsistenten Datenkennzeichnungen werden dem Experten zur Neuklassifizierung gegeben.

**Patentansprüche**

1. Computer-implementiertes Verfahren zur Erzeugung eines trainierten probabilistischen graphischen Modells zur Überprüfung und/oder Verbesserung der Konsistenz von Datenkennzeichnungen bei der medizinischen Bildverarbeitung, wobei das Verfahren die folgenden Schritte umfasst:

   - Bereitstellen eines Satzes von Bildern als Trainingsdaten, wobei die Bilder Bildinformationen jeweils einer Zelle umfassen;
   - Errechnen eines Merkmalsraumes für die Trainingsdaten, vorzugsweise mit Hilfe eines Deep Convolutional Neural Network (DCNN), wobei ein Bild einem Merkmalsvektor im Merkmalsraum entspricht;
   - Errechnen der Ähnlichkeiten zwischen mindestens zwei Datenpunkten der Trainingsdaten auf Basis des Merkmalsraumes;
   - Bereitstellen von Experten-generierten Datenkennzeichnungen für die Zellen, deren Bilder als Trainingsdaten bereitgestellt wurden;
   - Errechnen von verborgenen Datenkennzeichnungen auf Basis der Ähnlichkeiten zwischen mindestens zwei Datenpunkten der Trainingsdaten und der Experten-generierten Datenkennzeichnungen;
   - Erzeugung eines probabilistischen graphischen Modells zur Anpassung der verborgenen Datenkennzeichnungen, wobei das probabilistische Modell vorzugsweise ein Conditional Random Field (CRF) Modell ist.

2. Computer-implementiertes Verfahren, wobei das probabilistische graphische Modell, wie in Anspruch 1 erhalten, zur Anpassung von verborgenen Datenkennzeichnungen bei der medizinischen Bildverarbeitung von Zellen verwendet wird, umfassend die Schritte:

   - Bereitstellen von verborgenen Datenkennzeichnungen auf Basis der Ähnlichkeiten zwischen mindestens zwei Datenpunkten von Bildern als Trainingsdaten und mit diesen Trainingsdaten assoziierten Experten-generierten Datenkennzeichnungen, wobei die Bilder Bildinformationen jeweils einer Zelle umfassen;
   - Verknüpfen der verborgenen Datenkennzeichnungen und der Experten-generierten Datenkennzeichnungen, wobei die Verknüpfung ein Verbinden aller verborgenen Datenkennzeichnungen untereinander mittels ungerichteter Kanten und ein Verbinden von verborgenen Datenkennzeichnungen und Experten-generierten Datenkennzeichnungen mittels gerichteter Kanten umfasst;
   - Anpassen oder Bestätigen der verborgenen Datenkennzeichnungen auf Basis des probabilistischen graphischen Modells.

3. Computer-implementiertes Verfahren zur Erzeugung eines trainierten probabilistischen graphischen Modells zur Überprüfung und/oder Verbesserung der Konsistenz von Datenkennzeichnungen bei der medizinischen Bildverarbeitung, wobei die Zelle verschiedene Entwicklungsstufen einer Zelle repräsentieren kann, umfassend die folgenden Schritte:

- Bereitstellen eines Satzes von Bildern als Trainingsdaten, wobei die Bilder Bildinformationen jeweils einer Zelle umfassen;
- Errechnen eines Merkmalsraumes für die Trainingsdaten, vorzugsweise mit Hilfe eines Deep Convolutional Neural Network (DCNN), wobei ein Bild einem Merkmalsvektor im Merkmalsraum entspricht;
- Errechnen der Ähnlichkeiten zwischen mindestens zwei Datenpunkten der Trainingsdaten auf Basis des Merkmalsraumes;
- Errechnen einer Pseudozeit, welche die Datenpunkte gemäß einer Entwicklungsabfolge ordnet;
- Bereitstellen von Experten-generierten Datenkennzeichnungen für die Zellen, deren Bilder als Trainingsdaten bereitgestellt wurden;
- Errechnen von verborgenen Datenkennzeichnungen auf Basis der in der Pseudozeit reflektierten Entwicklungsabfolge und der Experten-generierten Datenkennzeichnungen;
- Erzeugung eines probabilistischen graphischen Modells zur Anpassung der verborgenen Datenkennzeichnungen, wobei vorzugsweise das probabilistische Modell bei einer linearen Entwicklungsabfolge ein Hidden Markov Modell (HMM) und bei einer dichotomen Entwicklungsabfolge ein Hidden Markov Baum (HMT)ist.

4. Computer-implementiertes Verfahren, wobei das probabilistische graphische Modell, wie in Anspruch 3 erhalten, zur Anpassung von verborgenen Datenkennzeichnungen bei der medizinischen Bildverarbeitung von Zellen, welche verschiedene Zell-Entwicklungsstufen repräsentieren können, verwendet wird, umfassend die Schritte:

- Bereitstellen von verborgenen Datenkennzeichnungen auf Basis der Ähnlichkeiten zwischen mindestens zwei Datenpunkten eines Satzes von Bildern als Trainingsdaten, wobei die Bilder Bildinformationen jeweils einer Zelle umfassen, und einer daraus abgeleiteten Pseudozeit, sowie von mit den Trainingsdaten assoziierten Experten-generierten Datenkennzeichnungen;
- Verknüpfen der verborgenen Datenkennzeichnungen und der Experten-generierten Datenkennzeichnungen, wobei die Verknüpfung ein Verbinden aller Datenkennzeichnungen mittels gerichteter Kanten umfasst;
- Anpassen oder Bestätigen der verborgenen Datenkennzeichnungen auf Basis des probabilistischen graphischen Modells.

5. Computer-implementiertes Verfahren nach Anspruch 2 oder 4, wobei die angepassten oder bestätigten verborgenen Datenkennzeichnungen, welche auf Basis des probabilistischen graphischen Modells gem. Anspruch 2 oder 4 erhalten werden, für ein erneutes Durchlaufen des Verfahrens gem. Anspruch 2 oder Anspruch 4 verwendet werden.

6. Computer-implementiertes Verfahren nach einem der Ansprüche 1 oder 3, wobei die Errechnung der Ähnlichkeiten zwischen mindestens zwei Datenpunkten die Erstellung eines Ähnlichkeitsgraphen umfasst.

7. Computer-implementiertes Verfahren nach einem der Ansprüche 2 oder 4, wobei das Verknüpfen der errechneten Datenkennzeichnung und der Experten-generierten Datenkennzeichnung auf Basis der bedingten Wahrscheinlichkeit der Korrektheit der Experten-generierten Datenkennzeichnung durchgeführt wird.

8. Datenverarbeitungsvorrichtung zur Erzeugung eines trainierten probabilistischen graphischen Modells zur Überprüfung und/oder Verbesserung der Konsistenz von Datenkennzeichnungen bei der medizinischen Bildverarbeitung, umfassend:

- eine Einheit zum Bereitstellen eines Satzes von Bildern als Trainingsdaten, wobei die Bilder Bildinformationen jeweils einer Zelle umfassen;
- eine Einheit zum Errechnen eines Merkmalsraumes für die Trainingsdaten;
- eine Einheit zur Errechnung der Ähnlichkeiten zwischen mindestens zwei Datenpunkten auf Basis des Merkmalsraumes;
- optional eine Einheit zur Errechnung einer Pseudozeit, welche die Datenpunkte gemäß einer Entwicklungsabfolge ordnet;
- eine Einheit zum Bereitstellen von Experten-generierten Datenkennzeichnungen für die Zellen, deren Bilder als Trainingsdaten bereitgestellt wurden;
- eine Einheit zur Errechnung von verborgenen Datenkennzeichnungen auf Basis (i) der Ähnlichkeiten zwischen mindestens zwei Datenpunkten oder auf Basis der in der Pseudozeit reflektierten Entwicklungsabfolge und (ii) der Experten-generierten Datenkennzeichnungen;

- eine Einheit zur Erzeugung eines probabilistischen graphischen Modells zur Anpassung der verborgenen Datenkennzeichnungen.

9. Datenverarbeitungsvorrichtung zur Anpassung von verborgenen Datenkennzeichnungen bei der medizinischen Bildverarbeitung von Zellen auf Basis eines trainierten probabilistischen graphischen Modells, umfassend:

- eine Einheit zum (i) Bereitstellen von verborgenen Datenkennzeichnungen auf Basis der Ähnlichkeiten zwischen mindestens zwei Datenpunkten eines Satzes von Bildern als Trainingsdaten und mit diesen Trainingsdaten assoziierten Experten-generierten Datenkennzeichnungen, wobei die Bilder Bildinformationen jeweils einer Zelle umfassen, oder (ii) zum Bereitstellen von verborgenen Datenkennzeichnungen auf Basis der Ähnlichkeiten zwischen mindestens zwei Datenpunkten eines Satzes von Bildern als Trainingsdaten, wobei die Bilder Bildinformationen jeweils einer Zelle umfassen, und einer daraus abgeleiteten Pseudozeit, sowie von mit den Trainingsdaten assoziierten Experten-generierten Datenkennzeichnungen;
- eine Einheit (i) zum Verknüpfen der verborgenen Datenkennzeichnungen und der Experten-generierten Datenkennzeichnungen, wobei die Verknüpfung ein Verbinden aller verborgenen Datenkennzeichnungen untereinander mittels ungerichteter Kanten und ein Verbinden von verborgenen Datenkennzeichnungen und Experten-generierten Datenkennzeichnungen mittels gerichteter Kanten umfasst, oder (ii) zum Verknüpfen der verborgenen Datenkennzeichnungen und der Experten-generierten Datenkennzeichnungen, wobei die Verknüpfung ein Verbinden aller Datenkennzeichnungen mittels gerichteter Kanten umfasst;
- eine Einheit zum Erhalten von angepassten verborgenen Datenkennzeichnungen auf Basis des probabilistischen graphischen Modells.

10. Verwendung eines trainierten probabilistischen graphischen Modells, welches gemäß einem Verfahren nach einem der Ansprüche 1, 3 oder 6 bereitgestellt wurde, zur Überprüfung und/oder Verbesserung der Konsistenz von Datenkennzeichnungen zur Überprüfung und/oder Verbesserung der Konsistenz von Datenkennzeichnungen bei der medizinischen Bildverarbeitung, wobei vorzugsweise das trainierte probabilistische graphische Modell verwendet wird, um auf Basis einer abgeleiteten verborgenen Datenkennzeichnung eine Experten-generierte Datenkennzeichnung von Bildinformationen zu korrigieren oder zu bestätigen.

11. Computer-implementiertes Verfahren zur Überprüfung und/oder Verbesserung der Konsistenz von Datenkennzeichnungen bei der medizinischen Bildverarbeitung, umfassend die Schritte:

- Bereitstellen des Bildes einer Zelle;
- Bereitstellen einer vorhandenen Experten-generierten Datenkennzeichnung;
- Errechnen eines Merkmalsraumes für das Bild;
- Anwenden eines Verfahrens auf Basis eines trainierten probabilistischen graphischen Modells auf die errechneten Merkmale des Bildes;
- Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
- Ausgabe und/oder Speicherung des Bildes zusammen mit der korrigierten oder bestätigten verknüpften Datenkennzeichnung.

12. Computer-implementiertes Verfahren nach Anspruch 11, wobei das trainierte probabilistische graphische Modell gemäß einem Verfahren nach einem der Ansprüche 1, 3 oder 6 bereitgestellt wurde und/oder das anzuwendende Verfahren ein Verfahren nach einem der Ansprüche 2, 4 oder 7 ist.

13. Computer-implementiertes Verfahren nach Anspruch 11 oder 13, umfassend die Schritte:

- Bereitstellen des Bildes einer Zelle;
- Bereitstellen einer vorhandenen Experten-generierten Datenkennzeichnung;
- Verwenden von Hintergrundinformationen zum Bild der Zelle, um die Topologie eines Hidden Markov Baumes (HMT), eine Startwahrscheinlichkeit und eine Emissionsmatrix zu definieren;
- Lernen der Merkmalsdarstellungen des Bildes unter Verwendung eines neuronales Netzwerkes;
- Wählen eines geeigneten Pseudozeit-Inferenzalgorithmus und Berechnen der Pseudozeiten anhand der Merkmalsvektoren;
- Sortieren der Experten-generierten Datenkennzeichnungen nach aufsteigender Pseudozeit;
- Einrichten eines Hidden Markov Baumes (HMT), bei dem die sortierten Experten-generierten Datenkennzeichnungen die beobachteten Informationen sind;

- Lernen der Parameter von Übergangsmatrizen unter Verwendung des verallgemeinerten EM-Algorithmus;
- Anwenden des verallgemeinerten Viterbi-Algorithmus, um die wahrscheinlichsten wahren Datenkennzeichnungen abzuleiten;
- Identifizieren von Bildern mit inkonsistenten Datenkennzeichnungen, indem die tatsächlichen Datenkennzeichnungen mit den Experten-generierten Datenkennzeichnungen verglichen werden;
- Ausgabe von Bildern mit inkonsistenten Datenkennzeichnungen, die zusätzlich vorgeschlagene Datenkennzeichnungen enthalten.

14. Computer-implementiertes Verfahren nach Anspruch 11 bis 13, wobei eine Korrektur einer bereits vorhandenen Experten-generierten Datenkennzeichnung zu einer Feedbackanfrage bei einem Experten bezüglich der Datenkennzeichnungsdiskrepanz führt.

15. Computer-implementiertes Verfahren nach Anspruch 11, 12 oder 14, wobei das Verfahren mit einem Bild einer Zelle und einer damit verknüpften korrigierten oder bestätigten verknüpften Datenkennzeichnung welche gem. Anspruch 11 ausgegeben wird, einmal oder mehrmals erneut durchlaufen wird.

16. Datenverarbeitungsvorrichtung die ausgebildet ist, die Konsistenz von Datenkennzeichnungen bei der medizinischen Bildverarbeitung zu überprüfen und/oder zu verbessern, wobei die Vorrichtung mindestens einen Prozessor und einen Speicher aufweist, wobei der mindestens eine Prozessor eingerichtet ist, um einen Programmcode aus dem Speicher zu laden und auszuführen und basierend auf dem Ausführen des Programmcodes die folgenden Schritte auszuführen:

- Bereitstellen des Bildes einer Zelle;
- Bereitstellen einer vorhandenen Experten-generierten Datenkennzeichnung für dieses Bild;
- Errechnen eines Merkmalsraumes für das Bild;
- Anwenden eines Verfahrens, vorzugsweise eines Verfahrens nach einem der Ansprüche 2, 4 oder 7, auf Basis eines trainierten probabilistischen graphischen Modells auf die errechneten Merkmale des Bildes, vorzugsweise auf Basis eines trainierten probabilistischen graphischen Modell das gemäß einem Verfahren nach einem der Ansprüche 1, 3 oder 6 bereitgestellt wurde;
- Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
- Ausgabe und/oder Speicherung des Bildes zusammen mit der korrigierten oder bestätigten verknüpften Datenkennzeichnung.

17. Computerprogrammprodukt mit einem Computerprogramm, welches direkt in eine Speichereinrichtung eines Computers ladbar ist, mit Programmabschnitten, um alle Schritte eines Verfahrens nach einem der Ansprüche 1 bis 7 oder 11 bis 15 auszuführen, wenn das Computerprogramm in dem Computer ausgeführt wird.

18. Computerlesbares Medium, auf welchem von einem Computer einlesbare und ausführbare Programmabschnitte gespeichert sind, um alle Schritte eines Verfahrens nach einem der Ansprüche 1 bis 7 oder 11 bis 15 auszuführen, wenn die Programmabschnitte von dem Computer ausgeführt werden.

# FIG 1

# FIG 2

# FIG 3

FIG 4

FIG 5

EP 3 736 817 A1

FIG 6

FIG 7

EP 3 736 817 A1

FIG 8

## FIG 9

81

21 23 25 27

36 91 36 36 36

22 24 26 28

## FIG 10

81

102

101 21 23 25 27

22 24 26 28

## FIG 11

81

23 25 27

21

36 36 36 36

22 24 111 26 28

112

| | Europäisches Patentamt<br>European Patent Office<br>Office européen des brevets | **EUROPÄISCHER TEILRECHERCHENBERICHT**<br>nach Regel 62a und/oder 63 des Europäischen Patent-<br>übereinkommens. Dieser Bericht gilt für das weitere<br>Verfahren als europäischer Recherchenbericht. | **Nummer der Anmeldung**<br>EP 20 16 2867 |

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| A | Boqian Wu ET AL: "Multi-Scale Deep Neural Network for Mitosis Detection in Breast Cancer Histological Images",<br><br>,<br>9. November 2017 (2017-11-09), XP055623715,<br>DOI: 10.20944/preprints201711.0063.v1<br>Gefunden im Internet:<br>URL:https://www.preprints.org/manuscript/201711.0063/v1<br>[gefunden am 2019-09-19]<br>* Zusammenfassung *<br>* Kapitel "3.1 Dataset";<br>Kapitel "2.2 Multi-scale Fused Fully Convolutional Network"; Kapitel "2.3 FF-CNN+CRF model" *<br>-----<br><br>-/-- | 11-18 | INV.<br>G16H50/70<br>G16H30/40 |

**RECHERCHIERTE SACHGEBIETE (IPC)**

G16H

## UNVOLLSTÄNDIGE RECHERCHE

Die Recherchenabteilung ist der Auffassung, daß ein oder mehrere Ansprüche, den Vorschriften des EPÜ nicht entspricht bzw. entsprechen, so daß nur eine Teilrecherche (R.62a, 63) durchgeführt wurde.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| München | 5. Oktober 2020 | Heidrich, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04E09)

Seite 1 von 4

Europäisches
Patentamt

European
Patent Office

Office européen
des brevets

**EUROPÄISCHER
TEILRECHERCHENBERICHT**

Nummer der Anmeldung

EP 20 16 2867

## EINSCHLÄGIGE DOKUMENTE

| Kategorie | Kennzeichnung des Dokuments mit Angabe, soweit erforderlich der maßgeblichen Teile | Betrifft Anspruch |
|---|---|---|
| A | YIHUA CHEN ET AL: "Similarity-based Classification: Concepts and Algorithms", JOURNAL OF MACHINE LEARNING RESEARCH, MIT PRESS, CAMBRIDGE, MA, US, Bd. 10, 31. März 2009 (2009-03-31), Seiten 747-776, XP058264234, ISSN: 1532-4435 * das ganze Dokument, inbesondere Abstract und Kapitel "I. Introduction" * ----- | 11-18 |
| A | FILIPPO PICCININI ET AL: "Advanced Cell Classifier: User-Friendly Machine-Learning-Based Software for Discovering Phenotypes in High-Content Imaging Data", CELL SYSTEMS, Bd. 4, Nr. 6, 28. Juni 2017 (2017-06-28), Seiten 651-655.e5, XP055624626, US ISSN: 2405-4712, DOI: 10.1016/j.cels.2017.05.012 * das ganze Dokument, insbesondere Seite 1, "Highlights" * ----- | 11-18 |
| A | AN-AN LIU ET AL: "A Semi-Markov Model for Mitosis Segmentation in Time-Lapse Phase Contrast Microscopy Image Sequences of Stem Cell Populations", IEEE TRANSACTIONS ON MEDICAL IMAGING, IEEE SERVICE CENTER, PISCATAWAY, NJ, US, Bd. 31, Nr. 2, 26. September 2011 (2011-09-26), Seiten 359-369, XP011491026, ISSN: 0278-0062, DOI: 10.1109/TMI.2011.2169495 * das ganze Dokument, insbesondere Abstract; S. 360, Abschnitt "B. Our Approach"; Fig. 1 * ----- | 11-18 |

-/--

**KLASSIFIKATION DER ANMELDUNG (IPC)**

**RECHERCHIERTE SACHGEBIETE (IPC)**

EPO FORM 1503 03.82 (P04C12)

Europäisches
Patentamt

European
Patent Office

Office européen
des brevets

**EUROPÄISCHER
TEILRECHERCHENBERICHT**

Nummer der Anmeldung

EP 20 16 2867

| | **EINSCHLÄGIGE DOKUMENTE** | | KLASSIFIKATION DER ANMELDUNG (IPC) |
|---|---|---|---|
| Kategorie | Kennzeichnung des Dokuments mit Angabe, soweit erforderlich der maßgeblichen Teile | Betrifft Anspruch | |
| A | NIE WEIZHI ET AL: "Modeling Temporal Information of Mitotic for Mitotic Event Detection", IEEE TRANSACTIONS ON BIG DATA, IEEE, Bd. 3, Nr. 4, 19. Juli 2017 (2017-07-19), Seiten 458-469, XP011673467, DOI: 10.1109/TBDATA.2017.2723395 * das ganze Dokument, insbesondere Abstract, Fig. 2 * ----- | 11-18 | |
| A | US 2011/243417 A1 (MADABHUSHI ANANT [US] ET AL) 6. Oktober 2011 (2011-10-06) * Anspruch 1 * ----- | 11-18 | |
| A | LIANG-CHIEH CHEN ET AL: "DeepLab: Semantic Image Segmentation with Deep Convolutional Nets, Atrous Convolution, and Fully Connected CRFs", ARXIV.ORG, CORNELL UNIVERSITY LIBRARY, 201 OLIN LIBRARY CORNELL UNIVERSITY ITHACA, NY 14853, 2. Juni 2016 (2016-06-02), XP080705599, * das ganze Dokument, insbesondere Abstract und Fig. 1 * ----- | 11-18 | **RECHERCHIERTE SACHGEBIETE** (IPC) |
| A | LIU YIMING ET AL: "Automatic Segmentation of Cervical Nuclei Based on Deep Learning and a Conditional Random Field", IEEE ACCESS, Bd. 6, 19. September 2018 (2018-09-19), Seiten 53709-53721, XP011692996, DOI: 10.1109/ACCESS.2018.2871153 * das ganze Dokument, insbesondere Abstract und Fig. 1 * ----- -/-- | 11-18 | |

EPO FORM 1503 03.82 (P04C12)

Seite 3 von 4

Europäisches Patentamt

European Patent Office

Office européen des brevets

**EUROPÄISCHER TEILRECHERCHENBERICHT**

Nummer der Anmeldung

EP 20 16 2867

| | **EINSCHLÄGIGE DOKUMENTE** | | KLASSIFIKATION DER ANMELDUNG (IPC) |
|---|---|---|---|
| Kategorie | Kennzeichnung des Dokuments mit Angabe, soweit erforderlich der maßgeblichen Teile | Betrifft Anspruch | |
| A | ALBAYRAK ABDULKADIR ET AL: "Mitosis detection using convolutional neural network based features", 2016 IEEE 17TH INTERNATIONAL SYMPOSIUM ON COMPUTATIONAL INTELLIGENCE AND INFORMATICS (CINTI), IEEE, 17. November 2016 (2016-11-17), Seiten 335-340, XP033060964, DOI: 10.1109/CINTI.2016.7846429 * das ganze Dokument insbesondere Abstract und Fig. 3 *<br>----- | 11-18 | |
| X | US 2012/269436 A1 (MENSINK THOMAS [FR] ET AL) 25. Oktober 2012 (2012-10-25) * Absätze [0036], [0062], [0085], [0166]; Abbildungen 2, 4 *<br>----- | 11-18 | **RECHERCHIERTE SACHGEBIETE** (IPC) |
| T | YUSHAN LIU ET AL: "TIMELY: Improving Labeling Consistency in Medical Imaging for Cell Type Classification", ARXIV.ORG, CORNELL UNIVERSITY LIBRARY, 201 OLIN LIBRARY CORNELL UNIVERSITY ITHACA, NY 14853, 10. Juli 2020 (2020-07-10), XP081719307, * das ganze Dokument *<br>----- | 11-18 | |

EPO FORM 1503 03.82 (P04C12)

1

Seite 4 von 4

Europäisches
Patentamt

European
Patent Office

Office européen
des brevets

**UNVOLLSTÄNDIGE RECHERCHE
ERGÄNZUNGSBLATT C**

**Nummer der Anmeldung**

EP 20 16 2867

---

Vollständig recherchierbare Ansprüche:
    11, 13-15

Unvollständig recherchierte Ansprüche:
    12, 16-18

Nicht recherchierte Ansprüche:
    1-10

Grund für die Beschränkung der Recherche:

Der Anmelder wurde mit Schreiben vom 29.07.2020 gemäß Regel 62a (1) EPÜ aufgefordert, die Regel 43 (2) EPÜ entsprechenden Patentansprüche anzugeben, auf deren Grundlage die Recherche durchzuführen ist.
Grund für die Aufforderung ist, dass die ursprünglich eingereichte Fassung mehrere unabhängige Ansprüche in der gleichen Kategorie enthält. Nach Artikel 84 in Verbindung mit Regel 43 (2) EPÜ darf eine Anmeldung nur dann mehr als einen unabhängigen Patentanspruch in einer bestimmten Kategorie enthalten, wenn der beanspruchte Gegenstand unter eine der in Regel 43 (2) Buchstaben a, b oder c EPÜ genannten Ausnahmesituationen fällt. Aus folgendem Grund/folgenden Gründen ist dies bei der vorliegenden Anmeldung jedoch nicht der Fall:
Ansprüche 1 und 3 beziehen sich nicht
- auf mehrere miteinander in Beziehung stehende Verfahren
- auf verschiedene Verwendungen des Verfahrens
- Alternativlösungen für eine bestimmte Aufgabe
Somit beziehen sich Ansprüche 1 und 3 de facto auf ein und denselben Gegenstand und unterscheiden sich nur durch die abweichenden Definitionen des Gegenstands, für den Schutz begehrt wird, bzw. nur durch die für die Merkmale dieses Gegenstands verwendete Terminologie. Die Ansprüche erfüllen nicht eine der in Regel 43 (2) EPÜ genannten Ausnahmesituationen.
Ansprüche 2 und 4 sind als pseudo-abhängige Ansprüche formuliert. Sie enthalten zwar eine Referenz auf Anspruch 1 bzw. Anspruch 4, es ist jedoch nicht ersichtlich, dass Ansprüche 2 und 4 alle Merkmale der Ansprüche 1 oder 3 enthalten, d.h. die Bedingungen für eine echte Abhängigkeit erfüllen. Grund dafür ist, dass sich Ansprüche 2 und 4 zwar auf ein probabilistische Modell wie in Anspruch 1 oder 3 erhalten, beziehen, die Erzeugung des Modells jedoch in Anspruch 1 und 3 unabhängig von den weiteren Merkmalen dieser Ansprüche geschieht. Das heißt, dass sich die Ansprüche 2 und 4 nur auf diesen letzten Verfahrensschritt beziehen, der ohne erkennbaren Zusammenhang zu den vorherigen Verfahrensschritten in diesen Ansprüchen steht.
Demzufolge stellen Anspruch 2 und 4 in der gegenwärtigen Interpretation unabhängige Ansprüche dar.
Anspruch 11 ist ein weiterer Verfahrensanspruch, der in Verbindung mit Verfahrensansprüchen 1 und 3 ebenfalls unter keine der in Regel 43 (2) EPÜ genannten Ausnahmen fällt. Zunächst enthält Anspruch 11 keinen Verweis auf einen anderen unabhängigen Anspruch, sodass sich eine Beziehung zwischen Ansprüchen ableiten ließe. Darüber hinaus definiert er auch ein anderes (technisches) Problem welches zu lösen sei.
Ein Verweis auf andere unabhängige Ansprüche findet sich erst im abhängigen Anspruch 12, wobei dieser Kombinationen von Ansprüche abdeckt die Lichte der Kombination von unabhängigen Ansprüchen nicht sinnvoll

Europäisches
Patentamt

European
Patent Office

Office européen
des brevets

## UNVOLLSTÄNDIGE RECHERCHE
### ERGÄNZUNGSBLATT C

**Nummer der Anmeldung**

EP 20 16 2867

erscheinen, z.B. ein Modell aus Anspruch 1 (Datenkennzeichnungen) angewendet in einem Verfahren nach Anspruch 4 (Korrektur von verborgenen Datenkennzeichnungen).

Die gegen die Verfahrensansprüche erhobenen Einwände gelten gleichlautend auch gegen die korrespondierenden Systemansprüche 8 (korrespondierend zu 1 und 3), 9 (korrespondierend zu 3 und 11), 16 (korrespondierend zu 11). Ansprüche 8, 9, 16 fallen darüber hinaus ebenfalls in keine der in Regel 43 (2) EPÜ genannten Ausnahmesituationen.

Es erscheint möglich, die gemachten Einwände durch eine Änderung der Ansprüche zu überwinden. Eine abschließende Beurteilung ob die Erfordernisse der Regel 43 (2) EPÜ und ggfs. Artikel 82 EPÜ erfüllt sind, erfolgt im weiteren Prüfungsverfahren.

Die Recherche wurde auf den Gegenstand beschränkt, den der Anmelder in seinem Schreiben vom 20.08.2020 in Beantwortung der Aufforderung nach R. 62a (1) oder/und 63 (1) EPÜ angegeben hat, d.h. Verfahrenanspruch 11 und Vorrichtungsanspruch 16. Damit schließt die Recherche die abhängigen Ansprüche 12 bis 15 und die unabhängigen Ansprüche 17 und 18 mit ein insofern sie sich auf Anspruch 11 bzw. 16 beziehen.

**ANHANG ZUM EUROPÄISCHEN RECHERCHENBERICHT
ÜBER DIE EUROPÄISCHE PATENTANMELDUNG NR.**

EP 20 16 2867

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-10-2020

| Im Recherchenbericht angeführtes Patentdokument | Datum der Veröffentlichung | Mitglied(er) der Patentfamilie | Datum der Veröffentlichung |
|---|---|---|---|
| US 2011243417 A1 | 06-10-2011 | US 2011243417 A1<br>WO 2010027476 A1 | 06-10-2011<br>11-03-2010 |
| US 2012269436 A1 | 25-10-2012 | KEINE | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **MEER et al.** *J Clin Pathol,* 2007, vol. 60 (7), 838-839
- **REID ; WERNISCH.** *Bioinforamtics,* 2016, vol. 32 (19), 2973-2980
- **KONDO et al.** *Proceedings of the Eighth Workshop on Statistical Machine Translation,* 2013, 503-511
- **HAGHVERDI et al.** *Nature Methods,* 2016, vol. 13 (10), 845-848
- **SAELENS et al.** *Nature Biotechnology,* 2019, vol. 37, 547-554
- **CANNOODT.** *SCORPIUS improves trajectory inference and identifies novel modules in dendritic cell development,* 2016
- **CHEN et al.** *Nature Communications,* 2019, vol. 10 (1), 1903
- **ALBERGANTE et al.** *Robust and scalable learning of complex datase ttopologies via ElPiGraph,* 2018
- **ABKOWITZ et al.** *Nature Medicine,* 1996, vol. 2 (2), 190-197
- **DURAND et al.** *IEEE Transactions on Signal Processing,* 2004, vol. 52 (9), 2251-2560
- **SANCHEZ et al.** *Pattern Recognition Letters,* 1997, vol. 18 (11-13), 1179-1186
- **KOPLOWITZ ; BROWN.** *Pattern Recognition,* 1981, vol. 13 (3), 251-255
- **SAEZ et al.** *Journal of Medical Informatics & Technologies,* 2015, vol. 24, 123-130
- **NORTHCUTT et al.** *Confident learning: estimating uncertainty in dataset labels, arXiv:1911.00068v1,* 2019
- Learning with confident examples: rank pruning for robust classification with noisy labels. **NORTHCUTT et al.** Proceedings of the 33rd Conference on Uncertainty in Artificial Intelligence. AUAI Press, 2017
- **COIFMAN ; LAFON.** *Applied and Computational Harmonic Analysis,* 2006, vol. 21 (1), 5-30